(19) Europäisches Patentamt
European Patent Office
Office européen des brevets

(11) Veröffentlichungsnummer: **0 012 347**
**B1**

(12) **EUROPÄISCHE PATENTSCHRIFT**

(45) Veröffentlichungstag der Patentschrift: **28.07.82**

(21) Anmeldenummer: **79104933.1**

(22) Anmeldetag: **05.12.79**

(51) Int. Cl.³: **C 07 D 495/14**, A 61 K 31/435 // C07D333/54, C07D333/70, C07C79/36, C07C49/807

# (54) 7,8,9,10-Tetrahydrothieno(3,2-e)pyrido(4,3-b)indole, Verfahren zu ihrer Herstellung und diese enthaltende Arzneimittel.

(30) Priorität: **14.12.78 DE 2854014**

(43) Veröffentlichungstag der Anmeldung: **25.06.80 Patentblatt 80/13**

(45) Bekanntmachung des Hinweises auf die Patenterteilung: **28.07.82 Patentblatt 82/30**

(84) Benannte Vertragsstaaten: **AT BE CH DE FR GB IT LU NL SE**

(56) Entgegenhaltungen:
**FR-M-2 568**
**CHEMICAL ABSTRACTS, Band 77, Nr. 23, 04-12-1972, Seiten 408-409, Zusammenfassung 152028t. Columbus, Ohio, USA**
**N.M. SHARKOVA et al. «Indole derivatives. XL. Synthesis of new condensed indole systems».**

**Die Akte enthält technische Angaben, die nach dem Eingang der Anmeldung eingereicht wurden und die nicht in dieser Patentschrift enthalten sind.**

(73) Patentinhaber: **Troponwerke GmbH & Co. KG, Berliner Strasse 156, D-5000 Köln 80 (DE)**

(72) Erfinder: **Boltze, Karl-Heinz, Dr., Am Burgtor 23, D-5060 Bergisch-Gladbach 3 (DE)**
Erfinder: **Seidel, Peter-Rudolf, Dr., Guntherstrasse 58, D-5000 Köln 90 (DE)**
Erfinder: **Jacobi, Haireddin, Dr., Finkenweg 2, D-5653 Leichlingen (DE)**
Erfinder: **Schwarz, Helmut Heinrich, Dr., Nachtigallenstrasse 5, D-5060 Bergisch-Gladbach 1 (DE)**
Erfinder: **Schöllnhammer, Günter, Dr., Hackberg 21, D-5060 Bergisch-Gladbach 1 (DE)**
Erfinder: **Dell, Hans-Dieter, Dr., Kalmüntener Strasse 5, D-5060 Bergisch-Gladbach 2 (DE)**

(74) Vertreter: **Dill, Erwin, Dr. et al, c/o BAYER AG Zentralbereich Patente Marken und Lizenzen Bayerwerk, D-5090 Leverkusen 1 (DE)**

**EP 0 012 347 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zu ihrer Herstellung und diese enthaltende 7,8,9,10-Tetrahydrothieno[3,2-e]pyrido[4,3-b]indole, Arzneimittel

Die vorliegende Erfindung betrifft neue 7,8,9,-10-Tetrahydrothieno[3,2-e]pyrido[4,3-b]indole, ein Verfahren zu ihrer Herstellung sowie diese enthaltende Arzneimittel, insbesondere das zentrale Nervensystem beeinflussende Mittel.

Das erfindungsgemässe Ringsystem ist bisher in der Literatur nicht beschrieben worden. In Chemical Abstracts 77, 408-409 (1972), Zusammenfassung 152082t sind Indolderivate beschrieben, die an Stelle eines Thiophenringes einen Furanring besitzen. Eine Wirkung auf das zentrale Nervensystem, insbesondere eine antidepressive Wirkung dieser Verbindungen ist nicht bekannt. In dem französischen Patent Nr. 2568M werden Indolderivate mit kuagulationshindernder Wirkung beschrieben, die an Stelle des Thienoringes einen Halogenrest tragen. Bei Kenntnis dieses Standes der Technik konnte nicht erwartet werden, dass die neuen erfindungsgemässen Tetrahydrothieno-indolderivate eine vorteilhafte Wirkung auf das zentrale Nervensystem besitzen.

Gegenstand der Anmeldung sind 7,8,9-10-Tetrahydrothieno[3,2-e]pyrido[4,3-b]indole der allgemeinen Formel (I)

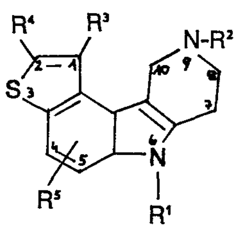

(I)

in welcher

$R^1$ für Wasserstoff, geradkettiges oder verzweigtes Alkyl mit 1 bis 4 Kohlenstoffatomen das gegebenenfalls durch Alkoxy mit 1 bis 2 Kohlenstoffatomen substituiert ist oder durch Alkylamino oder Dialkylaminoreste substituiert ist mit jeweils 1 bis 2 Kohlenstoffatomen pro Alkylrest oder für Phenyl-alkyl steht, wobei der Phenylrest gegebenenfalls substituiert ist durch Halogen oder

für Phenyl steht, wobei der Phenylrest gegebenenfalls durch Alkyl oder Alkoxyreste mit 1 bis 2 Kohlenstoffatomen substituiert ist oder gegebenenfalls durch Halogen oder durch Hydroxyl oder Trifluormethyl substituiert ist, oder

für eine Carbonylgruppe steht, die durch Alkyl mit 1 bis 4 Kohlenstoffatomen oder Phenyl substituiert ist, wobei die Phenylgruppe gegebenenfalls durch Nitro, Halogen oder Amino substituiert ist, oder

für eine Cycloalkylalkylgruppe mit 3 bis 6 Kohlenstoffatomen im Cycloalkylring und 1 bis 3 Kohlenstoffatomen in der Alkylkette steht,

$R^2$ für Wasserstoff, geradkettiges oder verzweigtes oder cyclisches Alkyl mit bis zu 6 Kohlenstoffatomen, wobei dieser Alkylrest gegebenenfalls substituiert ist durch Alkoxy mit 1 bis 2 Kohlenstoffatomen oder durch Alkylamino oder Dialkylaminoreste mit jeweils 1 bis 2 Kohlenstoffatomen im Alkylteil, oder für Phenyl steht, wobei der Phenylrest gegebenenfalls substituiert ist durch Alkylreste mit jeweils 1 bis 2 Kohlenstoffatomen oder durch Alkoxy mit 1 bis 2 Kohlenstoffatomen oder durch Halogen oder Trifluormethyl, oder

für Benzyl oder Phenethyl steht wobei der Phenylring gegebenenfalls substituiert ist durch Halogen, oder für Alkenyl mit bis zu 4 Kohlenstoffatomen steht, oder

für einen Alkoxycarbonylrest steht mit bis zu 4 Kohlenstoffatomen im Alkoxyrest, wobei dieser Alkoxyrest gegebenenfalls durch Dialkylaminogruppen mit jeweils 1 oder 2 Kohlenstoffatomen in der Alkylgruppe substituiert ist oder

für einen Benzoylalkylrest, wobei der Alkylrest bis zu 4 Kohlenstoffatome enthält und der Phenylrest gegebenenfalls durch Halogen substituiert ist, steht, oder

für einen Biphenylalkylrest steht mit 1 bis 4 Kohlenstoffatomen im Alkylrest, wobei der Phenylrest gegebenenfalls durch Halogen substituiert ist,

$R^3$ für Wasserstoff, geradkettiges oder verzweigtes Alkyl mit 1 bis 6 Kohlenstoffatomen, gegebenenfalls substituiert durch Halogen oder für einen gegebenenfalls durch Halogen oder Trifluormethyl substituierten Phenylrest steht,

$R^4$ für Wasserstoff, Alkyl mit 1 bis 4 Kohlenstoffatomen, insbesondere mit 1 bis 2 Kohlenstoffatomen, oder für eine Benzyloxycarbonylgruppe steht,

sowie gegebenenfalls mit Alkylhalogeniden gebildeten quarternären Salze oder ihre gegebenenfalls mit Basen oder Säuren gebildeten physiologisch verträglichen Salze.

Es wurde gefunden, dass man die 7,8,9,10--Tetrahydrothieno[3,2-e]pyrido[4,3-b]indole der allgemeinen Formel (I), in welcher $R^1$ bis $R^4$ die angegebene Bedeutung haben, erhält, wenn man

Hydrazinverbindungen der allgemeinen Formel (II)

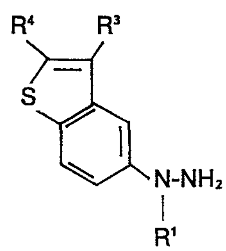

(II)

in welcher

$R^1$, $R^3$ und $R^4$ die oben angeführte Bedeutung haben, mit Piperidonen der allgemeinen Formel (III)

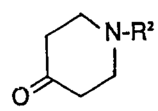

(III)

in welcher

$R^2$ die angeführte Bedeutung hat,

in Gegenwart von inerten organischen Lösungsmitteln bei Temperaturen zwischen 50 und 230°C und gegebenenfalls in Gegenwart von Kondensationsmitteln umsetzt und die so entstehenden Verbindungen der Formel (I) für den Fall, dass $R_1$ und $R_2$ Wasserstoffatome bedeuten, diese in bekannter Weise gegebenenfalls substituiert durch die für $R_1$ und $R_2$ obengenannten Reste und anschliessend die freien Verbindungen der Formel (I) gegebenenfalls in bekannter Weise in die quarternären oder die Additionssalze überführt.

Überraschenderweise zeigen die neuen erfindungsgemässen Verbindungen der allgemeinen Formel (I) ausgeprägte und vorteilhafte Wirkungen auf das zentrale Nervensystem und können beispielsweise als Antidepressiva dort Verwendung finden, wo bereits bekannte Antidepressiva unerwünschte Nebenwirkungen oder Gewöhnungssysteme zeigen. Sie stellen somit eine Bereicherung der Pharmazie dar.

Die Ausgangsverbindungen der allgemeinen Formel II und auch deren Vorstufen sind nur teilweise bekannt. Sie können nach an sich bekannten Methoden beispielhaft nach folgendem Reaktionsschema hergestellt werden:

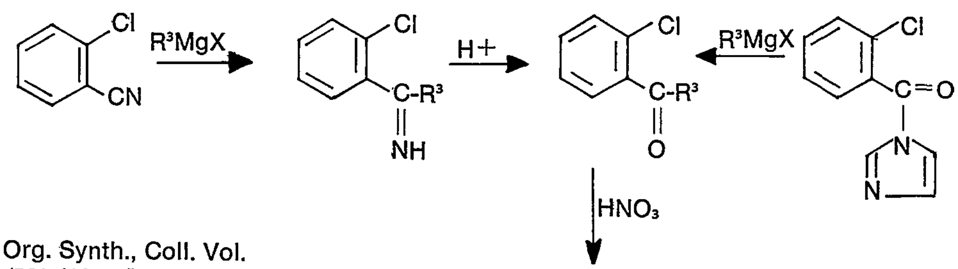


[vgl.: Org. Synth., Coll. Vol. III, 26, 562 (1955)]

[vgl.: H.A. Staab, u.a. Liebigs Ann. 655, 90 (1962)]

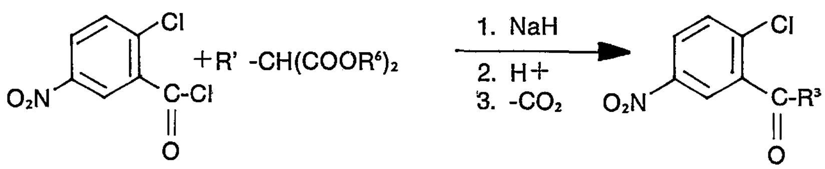


[vgl.: H. Henecka in Houben-Weyl Vol. 7/2b, Seite 1338 ff (1976)]

(R' = $R^3$ minus $CH_2$, soweit dies unter den als $R^3$ genannten Substituenten möglich ist).
($R^6$ = H; Alkyl, bevorzugt Methyl und Ethyl; tert. Butyl; Tetrahydropyranyl-(2)-; Benzyl).

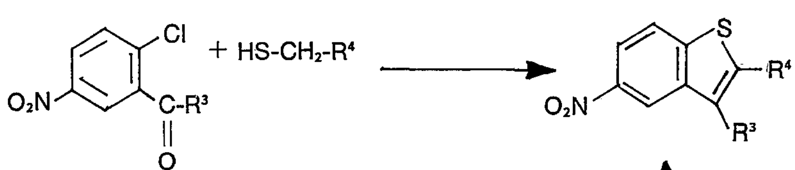

[vgl.: S. Rossi u. R. Trave Farmaco Ed. Sci. 15, 396 (1960) N.B. Chapman u.a., J. Chem. Soc. 1968, 518]

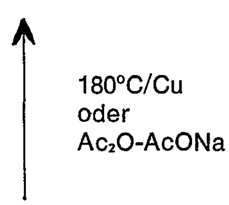


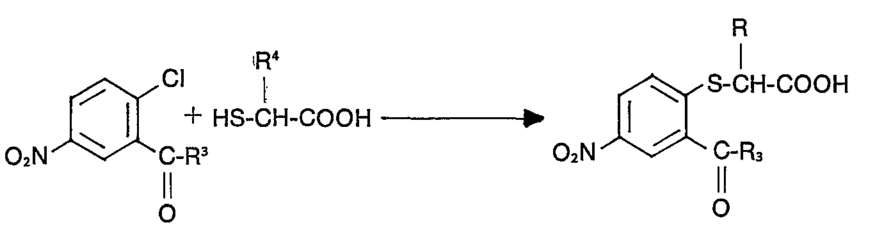

[vgl.: C. Angelini, Ann. Chim. (Rome) 47, 705 (1957)
A. Ricci u. N. Cagnoli, Ann. Chim. (Rome) 45, 172 (1955)
F. Sauter u. A. Dzerovicz, Monatsh. Chem. 101, 1806 (1970)]

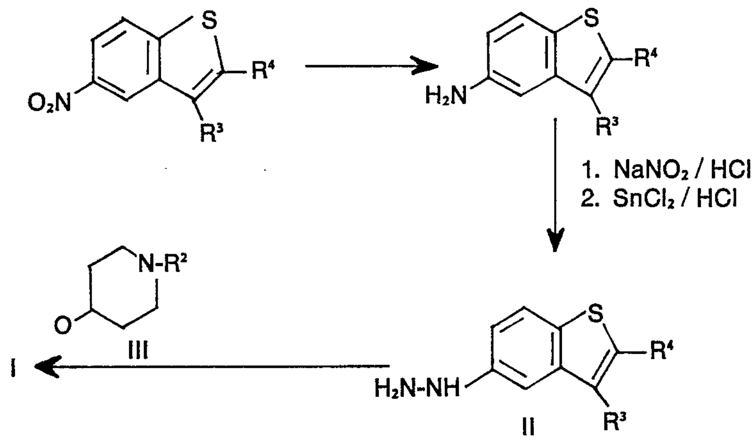


Bei der Reaktion:

II + III — — — — → I

kann man je nach der Reaktivität der Reaktionspartner grundsätzlich zwei verschiedene Reaktionsbedingungen unterscheiden:

1. Bei den säureunempfindlichen Reaktanten II oder III setzt man deren Salze, vorzugsweise die Hydrochloride, in einem geeigneten Verdünnungsmittel ein. Als Verdünnungsmittel können alle üblichen, für den Fischer-Indolringschluss

(a): E. Enders in Houben-Weyl, Band 10/2, Seite 546-586 (1967)

(b): A. Weissberger: The Chemistry of Heterocyclic Compounds. Indole Part I. Seite 232-317. Editor W. J. Houlihan, Wiley-Interscience 1972

geeignete Lösungsmittel verwendet werden. Beispielhaft seien genannt: Wasser, Methanol, Ethanol, Propyl- und insbesondere Isopropylalkohol, Benzol, Toluol, Xylol oder auch andere organische Lösungsmittel wie beispielsweise Dioxan, Eisessig, Polyphosphorsäureethylester oder hochsiedende Kohlenwasserstoffe. Als Kondensationsmittel können alle üblichen für den Fischer-Indolringsschluss geeigneten Katalysatoren (A. Weissberger: The Chemistry of Heterocyclic Compounds. Indole Part I, S. 246-258. Editor: W. J. Houlihan, Wiley-Interscience 1972) verwendet werden. Beispielhaft seien genannt: Zinkchlorid, Bortrifluorid, Bortrifluoridetherat, Chlorwasserstoff, konz. oder wasserfreie Schwefelsäure, Phosphorsäure, Polyphosphorsäure, Polyphosphorsäureethylester, Ameisensäure, saure Ionenaustauscher, z.B. Amberlite IR-120, oder Eisessig/Chlorwasserstoff. Bei empfindlicheren Reaktionspartnern II und III wird die Ringschlussreaktion zweckmässig in Gegenwart eines Inertgases, beispielsweise Stickstoff oder Argon, ausgeführt. Falls erforderlich kann zusätzlich Chlorwasserstoff als Kondensationsmittel eingeleitet werden.

Die Reaktionstemperaturen bei der Umsetzung von II und III können in einem grösseren Bereich variiert werden. Im allgemeinen arbeitet man zwischen 50° und 150°C, vorzugsweise zwischen 70° und 120°C. Die Reaktionszeiten bewegen sich im allgemeinen zwischen ½ und 20 Stunden. Die Umsetzung wird normalerweise bei normalem Druck durchgeführt.

Bei der Durchführung des erfindungsgemässen Verfahrens setzt man vorteilhaft auf 1 Mol der Hydrazinverbindung II das Piperidon der Formel III in einem leichten Überschuss von 0,1 bis 0,5 Mol ein.

Die Aufarbeitung erfolgt zweckmässig durch Eindampfen der Reaktionslösung, Aufnehmen des Konzentrats in einem geeigneten inerten organischen Lösungsmittel, Alkalisieren mit einer Base, z.B. NaOH oder $NH_3$ und Reinigen, gegebenenfalls mit Hilfe von Chromatographie an $SiO_2$ bzw. $Al_2O_3$.

Bisweilen hat es sich als zweckmässig erwiesen, anstelle des Ketons III das entsprechende Ethylenketal einzusetzen.

2. Bei Reaktanten (II oder III), die weniger leicht zur Kondensation neigen, oder die sich insbesondere durch Säureempfindlichkeit auszeichnen, arbeitet man vorzugsweise in hochsiedenden Lösungsmitteln. Für solche Lösungsmittel seien beispielhaft genannt: Dichlorbenzol, Trichlorbenzol, Ethylenglykol, Ethylenglykoldimethylether, wobei Ethylenglykol besonders bevorzugt ist. Die Reaktionstemperaturen können in einem grösseren Bereich variiert werden. Im allgemeinen arbeitet man zwischen 150° und 230°C, vorzugsweise zwischen 180° und 210°C, bei diesen Temperaturen zweckmässigerweise in Gegenwart von Inertgasen. Die Aufarbeitung erfolgt dann wie unter 1. beschrieben.

Verbindungen der Formel I, in der $R^1$ und/oder $R^2$ für Wasserstoff steht, können entsprechend substituiert werden indem man z.B. die

NH-Verbindung mit $NaNH_2$ oder vorzugsweise NaH in das Natriumsalz (I; $R^1$=Na) umwandelt und dieses in an sich bekannter Weise mit den entsprechend substituierten Halogeniden wie z.B. Alkylhalogeniden umsetzt.

Die Reaktion erfolgt zweckmässig in Gegenwart von Verdünnungsmitteln. Als Verdünnungsmittel kommen alle organischen inerten Lösungsmittel in Frage, Hierzu gehören vorzugsweise aromatische Kohlenwasserstoffe, wie z.B. Toluol, höhere Ether wie Ethylenglykoldimethylether und Dioxan, insbesondere aber aprotische Lösungsmittel, wie z.B. Dimethylformamid, Hexamethylposphorsäuretriamid, N-Methylpyrrolidon, Dimethylacetamid und Dimethylsulfoxid. Die Reaktionszeiten können in einem gewissen Bereich variiert werden. Im allgemeinen genügt 2- bis 6stündiges Erhitzen auf 40°-110°C, insbesondere auf 60 bis 80°C; in einzelnen Fällen genügt Raumtemperatur. Die Aufarbeitung erfolgt dann wieder analog wie oben beschrieben.

Als neue Wirkstoffe von besonderem Interesse seien die folgenden 7,8,9,10-Tetrahydrothieno-[3,2-e]pyrido[4,3-b]indole genannt:

9-Methyl-,7,8,9,10-Tetrahydrothienol[3,2-e]pyrido[4,3-b]indol, 9-Benzyl-, 1-Methyl-, 1,9-Dimethyl-, 1-Methyl-9-propyl-, 9-Benzyl-1-methyl-, 9--Diethylaminoethyl-1-methyl-, 9-Dimethylaminopropoxycarbonyl-1-methyl-, 9-Methyl-1-phenyl-, 2-Benzyloxycarbonyl-1-methyl-, 1,9-Dimethyl-2--benzyloxycarbonyl, 1-Butyl-9-methyl-, 9-sek.-Butyl-, 9-sek.-Butyl-1-methyl-, 9-Isopropyl-, 9--Isopropyl-1-methyl-, 1Butyl, 1-Butyl-9-isopropyl-, 9-Propyl-, 1-Butyl-9-benzyl-, 1-Phenyl-, 9-sek.-Butyl-1-phenyl-, 9-Benzyl-1-phenyl-, 9-[4-(4-Fluorphenyl)-4-oxobutyl]-1-methyl-, 9-[4,4-Bis-(4-fluorphenyl)butyl]-1-methyl-, 9-(3-Dimethylaminopropyl)-1-methyl-, 6-Propyl-, 9-Methyl-1-phenyl-6--propyl-, 6,9-Dibenzyl-1-methyl-, 6-(3-Dimethylaminopropyl)-1,9-dimethyl-, 9-Benzyl-6-(3-dimethylaminopropyl)-1-methyl-, 6-(3-Dimethylaminopropyl-1-methyl-, 9-Ethyl-, 6-Ethyl-9-methyl-, 6--Isobutyl-9-methyl-, 6-Cyclohexylmethyl-9-methyl-, 6-Ethyl-9-isopentyl-, 6-Phenyl-9-methyl-, 1--Methyl-9-ethyl-, 1-Methyl-9-butyl-, 1-Methyl-9--cyclohexyl-methyl-, 1-Methyl-9-cyclohexyl-, 1--Methyl-9-phenyl-, 1-Methyl-9-tert.-butyl-, 1-Methyl-9-isopentyl-, 1-Methyl-9-(2-methoxyethyl)-, 1--Methyl-9-(2-phenoxyethyl)-, 1-Methyl-9-(3-phenylpropyl)-, 1-Methyl-9-benzhydryl-, 1,6,9-Trimethyl-, 1,9-Dimethyl-6-ethyl-, 1,9-Dimethyl-6-(2-butyl)-, 1,9-Dimethyl-6-isopropyl-, 1,9-Dimethyl-6--butyl-, 1,9-Dimethyl-6-(2-methoxyethyl)-, 1,9-Dimethyl-6-benzhydryl-, 1,9-Dimethyl-6-[3-(p-fluorbenzoyl)-propyl]-, 1,9-Dimethyl-6-(3-dimethylamino-2-methylpropyl)-, 1-Methyl-6,9-bis(2-ethoxyäthyl)-, 1-Methyl-6-phenethyl-9-[2-(2-dimethylaminoethoxy)ethyl]-, 1-Methyl-6,9-bis(cyclohexylmethyl)-, 1-Methyl-6-ethyl-9-(4-methyl-1-piperazinylacetyl)-, 1-Methyl-6-ethyl-9-(1-methyl-4-piperidylcarbonyl)-, 1,6-Dimethyl-9-[1-(4-fluorphenyl)--4-piperidylcarbonyl]-, 1,9-Dimethyl-6-phenyl-, 1,9-Dimethyl-6-(4-fluorphenyl)-, 1-Ethyl-, 1-Ethyl--9-methyl-, 1,9-Diethyl-, 1-Ethyl-9-isopropyl-, 1--Ethyl-9-propyl-, 1-Ethyl-9-isobutyl-, 1-Ethyl-9-allyl-, 1-Ethyl-9-cyclohexyl-, 1-Ethyl-9-phenyl-, 1--Ethyl-9-(2-ethoxyäthyl)-, 1-Ethyl-9-(3-dimethylaminopropyl)-, 1-Ethyl-6,9-dimethyl-, 1-Ethyl-6--(2-methoxyethyl)-9-methyl-, 1-Ethyl-6-acetyl-9--methyl-, 1-Ethyl-6-(2-phenoxyethyl)-9-methyl-, 1--Ethyl-6-(4-chlorbenzyl)-9-methyl-, 1-Ethyl-6-(3--dimethylaminopropyl)-9-methyl-, 1,6-Diethyl-9--benzhydryl-, 1-Ethyl-6-(3-dimethylamino-2-methylpropyl)-9-benzhydryl-, 1-Ethyl-6-(2-ethoxyäthyl)-9-benzhydryl-, 1-Butyl-9-ethyl-, 1-Butyl-9--propyl-, 1-Butyl-9-cyclohexyl-, 1-Butyl-9-benzhydryl-, 1-Butyl-6,9-dimethyl-, 1-Butyl-6-isopropyl-9-methyl-, 1-Butyl-6-(2-ethoxyethyl)-9-methyl-, 1-Butyl-6-(3-dimethylamino-2-propyl)-9-methyl-, 1-Butyl-6-(3-dimethylamino-2-methylpropyl)-9--methyl-, 1-Butyl-6-(2-phenethyl)-9-ethyl-, 1-Butyl-6-benzhydryl-9-ethyl-, 1-Butyl-6-(3-phenylpropyl)-9-ethyl-, 1-Butyl-6-(4-chlorbenzyl)-9-ethyl-, 1-Butyl-6-(2-methoxyethyl)-9-ethyl-, 1-Isopentyl-, 1-Isopentyl-9-methyl-, 1-Isopentyl-9-ethyl-, 1-Isopentyl-9-isopropyl-, 1-Isopentyl-9-allyl-, 1-Isopentyl-9-cyclohexyl-, 1-Isopentyl-9-phenyl-, 1-Isopentyl-9-(1-methyl-4-piperidylcarbonyl)-, 1-Isopentyl--6,9-dimethyl-, 1-Isopentyl-6-(2-methoxyethyl)-9--methyl-, 1-Isopentyl-6-(3-dimethylaminopropyl)--9-methyl-, 1-Isopentyl-6-cinnamyl-9-methyl-, 1--Isopentyl-6-(2-phenoxyethyl)-9-(3-dimethylaminopropyl)-, 1-Isopentyl-6-cyclohexylmethyl-9-(3--dimethylaminopropyl)-, 1-(2-Cyclohexylethyl)-, 1-(2-Cyclohexylethyl)-9-methyl-, 1-(2-Cyclohexylethyl)-9-methyl-, 1-(2-Cyclohexylethyl)-9-ethyl-, 1-(2-Cyclohexylethyl)-9-isopropyl-, 1-(2-Cyclohexylethyl)-9-allyl-, 1-(2-Cyclohexylethyl)-2-cyclohexyl-, 1-(2-Cyclohexylethyl)-9-(1-methyl-4-piperidylcarbonyl)-, 1-(2-Cyclohexylethyl)-6,9-dimethyl-, 1-(2-Cyclohexylethyl)-6-phenyl-9-methyl-, 1--(2-Cyclohexylethyl)-9-[3-(4-methyl-1-piperazinyl--propyl]-, 1-Phenyl-9-ethyl-, 1-Phenyl-9-propyl-, 1-Phenyl-9-isopropyl-, 1-Phenyl-9-allyl-, 1-Phenyl-9-(2-phenethyl)-, 1-Phenyl-9-(2-ethoxyethyl)-, 1-Phenyl-9-(4-fluorphenyl)-4-oxobutyl)-, 1-Phenyl--6-propyl-9-methyl-, 1-Phenyl-6-cyclohexylmethyl--9-methyl-, 1-Phenyl-6-benzhydryl-9-methyl-, 1--Phenyl-6-(2-methoxyethyl)-9-methyl-, 1-Phenyl--6-(3-dimethylaminopropyl)-9-methyl-, 1-Phenyl--6-(3-phenylpropyl)-9-methyl-, 1-(2-Phenethyl)-, 1--(2-Phenethyl)-9-methyl-, 1-(2-Phenethyl)-9-isopropyl-,1-(2-Phenethyl)-9-allyl-, 1-(2-Phenethyl)-9--propyl-, 1-(2-Phenethyl)-9-cyclohexyl-, 1-(2--Phenethyl)-9-(2-ethoxyethyl)-, 1-(2-Phenethyl)-9--(1-methyl-4-piperidylcarbonyl)-, 1-(2-Phenethyl)--6,9-dimethyl-, 1-(2-Phenethyl)-6-(2-methoxyethyl)--9-methyl-, 1-(2-Phenethyl)-6-cyclohexylmethyl--9-methyl-, 1-(2-Phenethyl)-6-(3-dimethylaminopropyl)-9-methyl-, 1-(2-Phenethyl-6-(4-brombenzyl)-9-[2-(2-dimethylaminoethoxy)ethyl-, 1-(2--Phenethyl)-6-isobutyl-9-[2-(2-dimethylaminoethoxy)]-ethyl-, 2,9-Dimethyl-, 2-Methyl-9-ethyl-, 2--Methyl-9-cyclohexyl-, 2,6,9-Trimethyl-, 2,9-dimethyl-6-(4-fluorbenzyl)-, 2,9-Dimethyl-6-allyl-, 1,2--Dimethyl-, 1,2,9-Trimethyl-, 1,2-Dimethyl-9-allyl-, 1,2,6,9-Tetramethyl-, 1,2,9-Trimethyl-6-benzhydryl-, 1,2,9-Trimethyl-6-(2-ethoxyethyl)-, 1,2-Diphenyl-, 1,2-Diphenyl-9-methyl-, 1,2-Diphenyl-6,9-dimethyl-, 1,2-Diphenyl-6-(3-dimethylaminopropyl)-9-methyl-, 1-Methyl-2-phenyl-, 1,9-Dimethyl-2-phenyl-, 1-Methyl-2-phenyl-9-isobutyl-, 1-Methyl-2-phenyl-6-ethyl-9-isobutyl-, 1-Methyl-2-phenyl-6-ethyl-9-(3-dimethylaminopropyl)-, 1-Methyl-2-phenyl-6-(2-ethoxyethyl)-9-methyl-, 1-Ethyl-2-phenyl-9-methyl-, 1-Ethyl-2-phenyl-9-cyclohexyl-, 1-Ethyl-2-phenyl-6,9-bis(cyclohexylmethyl)-, 1-Ethyl-6-cyclohexylmethyl-9-methyl-, 2-Phenyl-9-methyl-, 2-Phenyl-6-(3-dimethylaminopropyl)-9-methyl-, 6,9-Bis(3-dimethylaminopropyl)-1-methyl-7,8,9,10-tetrahydrothieno[3,2-e]pyrido[4,3-b]-indol und 1,9-Dimethyl-7,8,9,10-tetrahydrothieno-[3,2-e]pyrido[4,3-b]indolmethojodid.

Von besonderem Interesse sind Verbindungen der allgemeinen Formel I in welcher

$R^1$ für Wasserstoff, geradkettiges oder verzweigtes Alkyl mit 1 bis 4 Kohlenstoffatomen das gegebenenfalls durch Alkoxy mit 1 bis 2 Kohlenstoffatomen substituiert ist, für Benzyl oder Phenyl steht, welche gegebenenfalls durch Fluor oder Chlor substituiert sind,

$R^2$ für Wasserstoff, geradkettiges oder verzweigtes oder cyclisches Alkyl mit bis zu 6 Kohlenstoffatomen, Phenyl oder Benzyl steht, oder für einen 3-(p-Fluorbenzoyl)-propyl- oder 4,4-Bis-(4-fluorphenyl)-butylrest steht,

$R^3$ für Wasserstoff, Alkyl mit 1 bis 2 Kohlenstoffatomen, Benzyl oder Phenäthyl steht, wobei die Alkyl- und Phenylreste gegebenenfalls durch Fluor oder Chlor substituiert sind, und

$R^4$ für Wasserstoff oder Alkyl mit 1 bis 2 Kohlenstoffatomen steht.

Zur vorliegenden Erfindung gehören pharmazeutische Zubereitungen, die neben nichttoxischen, inerten pharmazeutisch geeigneten Trägerstoffen einen oder mehrere erfindungsgemässe Wirkstoffe enthalten oder die aus einem oder mehreren erfindungsgemässen Wirkstoffen bestehen sowie Verfahren zur Herstellung dieser Zubereitungen.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragées, Kapseln, Pillen, Granulate, Suppositorien, Lösungen, Suspensionen und Emulsionen genannt.

Bisher nicht bekannte Ausgangsverbindungen

Beispiel A

3-Butyl-5-hydrazinobenzothiophenhydrochlorid

12,1 g 3-Butyl-5-aminobenzothiophenhydrochlorid werden mit 50 ml $H_2O$ und 50 ml konzentrierter HCl vermengt und bei $-5°C$ tropfenweise mit einer Lösung aus 3,6 g $NaNO_2$ in 40 ml Wasser versetzt. Diese Lösung wird in ein auf $-15°C$ gekühltes Gemisch aus 28,4 g $SnCl_2 \cdot 2H_2O$ und 50 ml konzentrierter HCl eingetropft. Nach Erwärmen auf Zimmertemperatur wird der ausgefallene Niederschlag abgesaugt, mit kalter konzentrierter HCl gewaschen, in Eiswasser gegeben, mit Ether überschichtet und mit 20%iger NaOH alkalisiert. Die etherische Phase wird gewaschen und getrocknet, mit HCl/Ether angesäuert; der Niederschlag in Ethylacetat aufgekocht und abgesaugt. Schmelzpunkt 168-170°C. Ausbeute: 10,0 g (78% d. Th.)

Beispiel B

3-Butyl-5-aminobenzothiophenhydrochlorid

38 g der entsprechenden 5-Nitro-Verbindung werden in 1,2 l Eethylacetat an 13 g Pd/C bei Zimmertemperatur hydriert (0,5 Std.). Anschliessend wird Pd/C abfiltriert, das Filtrat mit HCl/Ether angesäuert, der ausgefallene Niederschlag bei 20°C abgesaugt und in Ethylacetat aufgekocht. Schmelzpunkt 182-183°C.
Ausbeute: 74 g (95% der Theorie).

Beispiel C

3-Butyl-5-nitro-benzothiophen

5,6 g Cu werden unter $N_2$ 20 Minuten lang auf 210°C erhitzt und nach Abkühlen mit 110 g Chinolin und 27,9 g 3-Butyl-5-nitro-2-benzothiophencarbonsäure versetzt. Das Gemisch wird unter Rühren auf 180°C bis zur Beendigung der $CO_2$-Entwicklung erhitzt. Nach dem Erkalten wird die Reaktionsmasse mit $CH_2Cl_2$ abdekantiert, das Chinolin durch Ausschütteln mit 20%iger HCl ausgewaschen, die organische Phase gewaschen, getrocknet und an $Al_2O_3$ gereinigt. Schmelzpunkt 82-83°C.
Ausbeute: 21,7 g (92% der Theorie)

Beispiel D

3-Butyl-5-nitro-2-benzothiophencarbonsäure

Zu 31,7 g NaOH, gelöst in 360 ml 50%igem Ethanol, werden 107 g des entsprechenden Ethylesters zugesetzt und 2 Stunden lang gekocht. Nach dem Eindampfen wird der Rückstand in Wasser aufgenommen und angesäuert. Schmelzpunkt 238-240°C.
Ausbeute: 100,6 g (98% der Theorie)

Beispiel E

3-Butyl-5-nitro-2-benzothiophencarbonsäureethylester

12 g Na werden in 250 ml Ethanol gelöst; in diese Na-Ethylatlösung werden bei 20°C nacheinander 62,5 g Mercaptoessigsäureethylester und eine Lösung aus 132,5 g 2-Chlor-5-nitrovalerophenon in 680 ml Ethanol eingetropft. Nach 3stündigem Kochen und längerem Stehen bei 8°C wird der ausgefallene Niederschlag abfiltriert, in Chloroform aufgenommen, gewaschen und getrocknet. Nach Abdampfen des Lösungsmittels wird das Produkt aus Ethanol rekristallisiert. Schmelzpunkt 92-93°C.
Ausbeute: 90 g (56% der Theorie)

Beispiel F

2-Chlor-5-nitrovalerophenon

Zu 715 ml rauchender $HNO_3$ tropft man bei $-15°C$ 133 g 2-Chlorvalerophenon und schüttet das Gemisch auf 4 kg Eis. Anschliessend wird die wässrige Phase mit Chloroform extrahiert, die organische Phase gewaschen, getrocknet

und eingeengt. Die ölige Verbindung wurde als Rohprodukt weiterverarbeitet.
Ausbeute: 150 g (92% der Theorie)

Beispiel G
2-Chlorvalerophenon

In eine etherische Mischung von Butyl-MgBr (aus 205,5 g Butylbromid und 26,5 g Mg-Spänen) werden 68,8 g 2-Chlorbenzonitril in 800 ml Ether eingetropft. Das Gemisch wird 10 Stunden lang gekocht, anschliessend mit 600 ml ca. 20%iger HCl tropfenweise versetzt. Nach Zugabe von 600 ml Wasser wird die etherische Phase gewaschen, getrocknet, eingedampft und der Rückstand destilliert. Siedepunkt 138-140°C. $n_D^{20}$: 1,5203. Ausbeute: 84 g (85% der Theorie)

In Analogie zu dem beschriebenen 3-Butyl-5--hydrazinobenzothiophenhydrochlorid wurden aus den entsprechenden 5-Aminobenzothiophenhydrochloriden nachstehende Hydrazinobenzothiophenderivate hergestellt
5-Hydrazino-[Base Fp. 180-188° (Zers.), Hydrochlorid, Fp. 214°]
3-Methyl-5-hydrazino-(Base Fp. 83-87°, Hydrochlorid, Fp. 177°)
3-Ethyl-5-hydrazino-(Base Fp. 35-37°, Hydrochlorid, Fp. 188-190°)
3-Isopentyl-5-hydrazino-(Hydrochlorid, Fp. 172-174°C)
3-Phenethyl-5-hydrazino-[Hydrochlorid, Fp. 181-185° (Zers.)]
3-Cyclohexylethyl-5-hydrazino-,
2-Benzyloxycarbonyl-3-methyl-5-hydrazino-[Base, Fp. 113°, Hydrochlorid, Fp. 192° (Zers.)]
3-Phenyl-5-hydrazino-[Base Fp. 142-143°, Hydrochlorid, Fp. 175° (Z.)]
2-Methyl-5-hydrazino-, 1,2-Dimethyl-5-hydrazino-,
2-Phenyl-5-hydrazino-, 1,2-Diphenyl-5-hydrazinobenzothiophenhydrochlorid.

Beispiel 1
7,8,9,10-Tetrahydrothieno[3,2-e]pyrido[4,3-b]-indol

15 g 5-Hydrazinobenzothiophenhydrochlorid und 15 g 4-Piperidon werden in 450 ml Isopropanol 2 Stunden am Rückflusskühler gekocht. Nach dem Abkühlen wird der ausgefallene Niederschlag abfiltriert und in 2 n NaOH suspendiert. Die Suspension wird mit Dichlormethan unterschichtet und gerührt; dann werden die Schichten getrennt und die organische Phase gewaschen, getrocknet und eingedampft. Nach Rekristallisation aus Isopropanol erhält man 7,8,9,10--Tetrahydrothieno[3,2-e]pyrido[4,3-b]indol vom Schmelzpunkt 190°.
Ausbeute: 13 g (76% der Theorie)
$C_{13}H_{12}N_2S$ (228,321)
ber.: C 68,39% H 5,30% N 12,27% S 14,04%
gef.: C 68,54% H 5,32% N 12,17% S 14,07%
Schmelzpunkt des Lactats: 210-220°C (Zers.)

Beispiel 2
9-Methyl-7,8,9,10-tetrahydrothieno[3,2-e]pyrido-[4,3-b]indol

4 g 5-Hydrazinobenzothiophenhydrochlorid, in 100 ml Isopropanol suspendiert, werden unter $N_2$ mit 2,8 ml 1-Methyl-4-piperidonhydrochlorid vereinigt. Die Lösung wird zum Sieden gebracht, und in der Hitze mit 10 ml HCl-gesättigtem Isopropanol versetzt. Nach ½stündigem Kochen wird das Lösungsmittel abdestilliert, der Rückstand mit Ammoniak behandelt, der Niederschlag abfiltriert und aus Methanol rekristallisiert. Man erhält 9-Methyl-7,8,9,10-tetrahydrothieno[3,2-e]pyrido[4,3-b]indol vom Schmelzpunkt 189°C.
Ausbeute: 3,2 g (66% der Theorie)
$C_{14}H_{14}N_2S$ (242,348)
ber.: C 69,39% H 5,82% N 11,56% S 13,32%
gef.: C 69,43% H 5,90% H 11,58% S 13,30%
Schmelzpunkt des Lactats: 185-189°C.

Beispiel 3
9-Benzyl-7,8,9,10-tetrahydrothieno[3,2-e]pyrido-[4,3-b]indollactat

Die Verbindung entsteht analog der in Beispiel 2 beschriebenen aus 9,5 g 1-Benzyl-4-piperidonhydrochlorid und 10,0 g 5-Hydrazinobenzothiophenhydrochlorid. Die unbeständige Base wird in das Lactat umgewandelt. Schmelzpunkt 183°C.
Ausbeute: 6,0 g (29% der Theorie)
$C_{23}H_{24}N_2O_3S$ (408,527)
ber.: C 67,62% H 5,92% N 6,90% S 7,85%
gef.: C 67,48% H 5,98% N 6,94% C 7,76%

Beispiel 4
1-Methyl-7,8,9,10-tetrahydrothieno[3,2-e]pyrido-[4,3-b]indol

Die Verbindung entsteht analog der in Beispiel 2 beschriebenen aus 5 g 5-Hydrazino-3--methylbenzothiophenhydrochlorid und 5 g 4-Piperidonhydrochloridmonohydrat. Schmelzpunkt 234°C.
Ausbeute: 4,2 g (74% der Theorie)
$C_{14}H_{14}N_2S$ (242,348)
ber.: C 69,38% H 5,82% N 11,56% S 13,23%
gef.: C 69,47% H 5,85% N 11,54% S 13,17%

Beispiel 5
1,9-Dimethyl-7,8,9,10-tetrahydrothieno[3,2-e]pyrido[4,3-b]indol

15,75 g 5-Hydrazino-3-methylbenzothiophenhydrochlorid werden in 500 ml Wasser suspendiert, mit Dichlormethan unterschichtet und bei ca. 0°C mit 10,5 g 1-Methyl-4-piperidon versetzt. Anschliessend wird der Ansatz mit n NaOH auf pH 9,5 eingestellt und 30 Minuten gerührt. Die abgetrennte organische Phase wird mit gesättigter NaCl-Lösung gewaschen, getrocknet, mit 500 ml Ethylenglykol vermischt und das Dichlormethan im Vakuum bei 12 mm abdestilliert. Anschliessend wird ein Teil des Glykols bei 0,01 mm und 100°C abdestilliert und die eingeengte Lösung ½ Stunde auf 200°C (Badtemperatur) in einer $N_2$-Atmosphäre erhitzt. Die beim Erkalten ausfallenden Kristalle werden abfiltriert, gewaschen und aus Ethylacetat rekristallisiert. Schmelzpunkt 224-227°C.
Ausbeute: 10,2 g (54% der Theorie)

$C_{15}H_{16}N_2S$ (264,375)

| | | | | |
|---|---|---|---|---|
| ber.: | C 70,27% | H 6,29% | H 10,93% | S 12,51% |
| gef.: | C 70,30% | H 6,50% | N 10,93% | S 12,54% |

Schmelzpunkt des Lactats: 203°C
Schmelzpunkt des Hydrochlorids: 295°C (Zers.)
Schmelzpunkt des Maleats: 216°C (Zers.)
Schmelzpunkt des Tartrats: 275°C (Zers.)

Beispiel 6

1-Methyl-9-propyl-7,8,9,10-tetrahydrothieno[3,2-e]-pyrido[4,3-b]indol

Die Verbindung entsteht analog der in Beispiel 5 beschriebenen aus 2,15 g 5-Hydrazino-3-methylbenzothiophenydrochlorid und 1,9 g 1-Propyl-4-piperidon. Schelzpunkt 169°C.
Ausbeute: 0,4 g (14% der Theorie)
$C_{17}H_{20}N_2S$ (284,429)

| | | | | |
|---|---|---|---|---|
| ber.: | C 71,79% | H 7,09% | N 9,85% | S 11,27% |
| gef.: | C 71,66% | H 7,18% | N 9,86% | S 11,37% |

Schmelzpunkt des Lactats: 191-193°C.

Beispiel 7

9-Benzyl-1-methyl-7,8,9,10-tetrahydrothieno-[3,2-e]pyrido[4,3-b]indol

Die Verbindung entsteht aus 10,1 g 5-Hydrazino-3-methylbenzothiophen und 10 ml Benzyl-4-piperidon analog der in Beispiel 5 beschriebenen Verbindung. Schmelzpunkt 93°C.
Ausbeute: 10,1 g (60% der Theorie)
$C_{21}H_{20}N_2S$ (332,473)

| | | | | |
|---|---|---|---|---|
| ber.: | C 75,87% | H 6,06% | N 8,43% | S 9,64% |
| gef.: | C 75,92% | H 6,21% | N 8,33% | S 9,37% |

Beispiel 8

9-(3-Dimethylaminopropyl)-1-methyl-7,8,9,10-tetrahydrothieno[3,2-e]pyrido[4,3-b]indol

Die Verbindung entsteht analog der in Beispiel 5 beschriebenen aus 5-Hydrazino-3-methylbenzothiophenhydrochlorid und 8,5 ml 1-(3-Dimethylaminopropyl-4-piperidon. Schmelzpunkt 184°C.
Ausbeute: 5 g (40% der Theorie)
$C_{19}H_{25}N_3S$ (327,429)

| | | | | |
|---|---|---|---|---|
| ber.: | C 69,68% | H 7,70% | N 12,83% | S 9,79% |
| gef.: | C 69,53% | H 7,69% | N 12,89% | S 9,93% |

Schmelzpunkt des Hydrochlorids: 350°C

Beispiel 9

9-(3-Dimethylaminopropoxycarbonyl)-1-methyl-7,8,9,10-tetrahydrothieno[3,2-e]pyrido[4,3-b]-indol

Pyridinhydrochlorid (aus 5 ml Pyridin), 2,1 g 5-Hydrazino-3-methylbenzothiophenhydrochlorid und 2,9 g 1-(3-Dimethylaminopropoxycarbonyl)-4-piperidonethylenketal werden zunächst unter $N_2$ auf 100°C und nach 2 Stunden auf 120°C erhitzt. Nach dem Abkühlen wird der Ansatz mit NaOH auf pH 7,5 eingestellt, mit Dichlormethan extrahiert, gewaschen, getrocknet, eingeengt und an $Al_2O_3$ aus Dichlormethan/Methanol (99:1) chromatografiert: Schmelzpunkt 184°C.
Ausbeute: 0,35 g (9% der Theorie)
$C_{20}H_{25}N_3O_2S$ (387,51)

| | | | |
|---|---|---|---|
| ber.: | C 61,99% | H 6,50% | N 10,85% |
| gef.: | C 62,14% | H 6,64% | N 10,85% |

Beispiel 10

9-Methyl-1-phenyl-7,8,9,10-tetrahydrothieno-[3,2-e]pyrido[4,3-b]indol

Die Verbindung entsteht analog der in Beispiel 5 beschriebenen aus 14 g 5-Hydrazino-3-phenylbenzothiophenhydrochlorid und 6,2 g 1-Methyl-4-piperidon. Schmelzpunkt > 250°C (Zers.)
Ausbeute: 7,8 g (49% der Theorie)
$C_{20}H_{18}N_2S$ (318,45)

| | | | | |
|---|---|---|---|---|
| ber.: | C 75,44% | H 5,70% | N 8,80% | S 10,07% |
| gef.: | C 75,31% | H 5,71% | N 8,84% | S 9,95% |

Beispiel 11

2-Benzyloxycarbonyl-1-methyl-7,8,9,10-tetrahydrothiano[3,2-e]pyrido[4,3-b]indol

1 g 5-Hydrazino-3-methylbenzothiophen-2-carbonsäurebenzylesterhydrochlorid und 1 g 4-Piperidonhydrochloridmonohydrat werden unter $N_2$ in 50 ml Isopropanol 10 Stunden gekocht. Anschliessend wird das Lösungsmittel abgedampft, der Rückstand in Wasser aufgeschlämmt, mit $KHCO_3$ alakalische gemacht und die wässrige Suspension mit Ethylacetat extrahiert; der Extrakt wird gewaschen, getrocknet, abgedampft und der Rückstand aus Isopropanol rekristallisiert. Schmelzpunkt 178°C.
Ausbeute: 0,4 g (37% der Theorie)
$C_{22}H_{20}N_2O_2S$ (376,484)

| | | | | |
|---|---|---|---|---|
| ber.: | C 70,19% | H 5,35% | N 7,44% | S 8,52% |
| gef.: | C 70,31% | H 5,43% | N 7,43% | S 8,55% |

Beispiel 12

1,9-Dimethyl-2-benzyloxycarbonyl-7,8,9,10-tetrahydrothieno[3,2-e]pyrido[4,3-b]indol

4,5 g 5-Hydrazino-3-methylbenzothiophen-2-carbonsäurebenzylesterhydrochlorid werden in einem Gemisch aus HCl und Eisessig suspendiert; nach Zusatz von 3 ml 1-Methyl-4-piperidon wird das Reaktionsgemisch 3 Stunden gekocht. Anschliessend wird das Lösungsmittel abgedampft, der Rückstand auf pH 7,5 eingestellt und, wie unter Beispiel 11 beschrieben, aufgearbeitet. Schmelzpunkt 174°C.
Ausbeute: 1,3 g (26% der Theorie)
$C_{23}H_{22}N_2O_2S$ (390,511)

| | | | | |
|---|---|---|---|---|
| ber.: | C 70,74% | H 5,68% | N 7,17% | S 8,21% |
| gef.: | C 70,71% | H 5,78% | N 7,09% | S 8,11% |

Beispiel 13

1-Butyl-9-methyl-7,8,9,10-tetrahydrothieno[3,2-e]-pyrido[4,3-b]indol

Die Verbindung entsteht analog der in Beispiel 5 beschriebenen aus 25,6 g 3-Butyl-5-hydrazinobenzothiophenhydrochlorid und 13,5 g 1-Methyl-4-piperidon in Ethylenglykol, wobei man 30 Minuten auf 100° und anschliessend auf 180°C erhitzt. Schmelzpunkt 192-193°C.
Ausbeute: 18 g (60% der Theorie)
$C_{18}H_{22}N_2S$ (298,456)

| | | | | |
|---|---|---|---|---|
| ber.: | C 72,44% | H 7,43% | N 9,39% | S 10,74% |
| gef.: | C 72,27% | H 7,40% | N 9,51% | S 10,73% |

Schmelzpunkt des Lactats: Fp. 200-202°C
Schmelzpunkt des Malats: Fp. 228-229°C (Salz der Apfelsäure)

Beispiel 14

9-sek.-Butyl-7,8,9,10-tetrahydrothieno[3,2-e]pyrido[4,3-b]indollactat

Die Verbindung entsteht analog der in Beispiel 12 beschriebenen aus 10 g 5-Hydrazinobenzothiophenhydrochlorid und 10 g 1-sek.-Butyl-4-piperidon.

Durch Zugabe von Milchsäure in acetonischer Lösung erhält man das Lactat vom Schmelzpunkt 170-171°C.

Ausbeute: 13,5 g (72% der Theorie)

$C_{20}H_{26}N_2O_3S$ (374,510)

| | | | | |
|---|---|---|---|---|
| ber.: | C 64,14% | H 7,00% | N 7,48% | S 8,56% |
| gef.: | C 64,04% | H 6,83% | N 7,51% | S 8,55% |

Beispiel 15

9-sek.-Butyl-1-methyl-7,8,9,10-tetrahydrothieno-[3,2-e]pyrido[4,3-b]indol

11 g 5-Hydrazino-3-methylbenzothiophenhydrochlorid und 8,5 g 1-sek.-Butyl-4-piperidon werden in 35 ml Isopropanol gekocht und währenddessen Isopropanol/HCl zugetropft. Anschliessend wird eingedampft, der Rückstand in Wasser aufgenommen, alkalisch gemacht und mit Dichlormethan extrahiert. Nach dem Waschen der organischen Phase und Trocknen wird eingedampft und der Rückstand aus Cyclohexan rekristallisiert. Schmelzpunkt 136°-137°C.

Ausbeute: 7 g (46% der Theorie)

$C_{18}H_{22}N_2S$ (298,46)

| | | | | |
|---|---|---|---|---|
| ber.: | C 72,44% | H 7,43% | N 9,39% | S 10,74% |
| gef.: | C 72,37% | H 7,36% | N 9,39% | S 10,63% |

Schmelzpunkt des Lactats: 171-175°C.

Beispiel 16

9-Iisopropyl-7,8,9,10-tetrahydrothieno[3,2-e]pyrido[4,3-b]indol

Die Verbindung entsteht analog der in Beispiel 15 beschriebenen aus 10 g 5-Hydrazinobenzothiophenhydrochlorid und 10 g 1-Isopropyl-4-piperidon. Schmelzpunkt 197-199°C.

Ausbeute: 9,5 g (71% der Theorie)

$C_{16}H_{18}H_2S$ (270,402)

| | | | | |
|---|---|---|---|---|
| ber.: | C 71,07% | H 6,71% | N 10,36% | S 11,86% |
| gef.: | C 71,27% | H 6,83% | N 10,61% | S 11,78% |

Beispiel 17

9-Isopropyl-1-methyl-7,8,9,10-tetrahydrothieno-[3,2-e]pyrido[4,3-b]indol

Die Verbindung entsteht analog der in Beispiel 15 beschriebenen aus 11 g 5-Hydrazino-3--methylbenzothiophenhydrochlorid und 10 g 1--Isopropyl-4-piperidon. Schmelzpunkt 199°-203°C.

Ausbeute: 8 g (55% der Theorie)

$C_{17}H_{20}N_2S$ (284,429)

| | | | | |
|---|---|---|---|---|
| ber.: | C 71,79% | H 7,09% | N 9,85% | S 11,27% |
| gef.: | C 71,93% | H 7,23% | N 9,80% | S 11,27% |

Schmelzpunkt des Lactats: 203°C.

Beispiel 18

1-Butyl-7,8,9,10-tetrahydrothieno[3,2-e]pyrido-[4,3-b]indol

Die Verbindung entsteht analog der in Beispiel 15 beschriebenen aus 25,6 g 3-Butyl-5-hydrazinobenzothiophenhydrochlorid und 20 g 4-Piperidonhydrochloridmonohydrat. Schmelzpunkt 224-225°C.

Ausbeute: 18 g (63% der Theorie)

$C_{17}H_{20}N_2S$ (284-429)

| | | | | |
|---|---|---|---|---|
| ber.: | C 71,79% | H 7,09% | N 9,85% | S 11,27% |
| gef.: | C 71,91% | H 7,14% | N 9,86% | S 11,24% |

Schmelzpunkt des Lactats: 216-217°C.

Beispiel 19

1-Butyl-9-isopropyl-7,8,9,10-tetrahydrothieno-[3,2-e]pyrido[4,3-b]indol

Die Verbindung entsteht analog der in Beispiel 5 beschriebenen aus 25,6 g 3-Butyl-5-hydrazinobenzothiophenhydrochlorid und 17 g 1-Isopropyl-4-piperidon. Schmelzpunkt 157-158°C.

Ausbeute: 13 g (40% der Theorie)

$C_{20}H_{26}N_2S$ (326,510)

| | | | | |
|---|---|---|---|---|
| ber.: | C 73,57% | H 8,03% | N 8,58% | S 9,82% |
| gef.: | C 73,67% | H8,04% | N 8,61% | S 9,72% |

Schmelzpunkt des Lactats: 183-185°C.

Beispiel 20

9-Propyl-7,8,9,10-tetrahydrothieno[3,2-e]pyrido-[4,3-b]indol

Diese Verbindung entsteht aus 2 g 5-Hydrazinobenzothiophenhydrochlorid und 1,7 g 1-Propyl-4-piperidon analog der in Beispiel 15 beschriebenen. Schmelzpunkt: 143-144°C.

Ausbeute: 2,1 g (78% der Theorie)

$C_{16}H_{18}N_2S$ (270,402)

| | | | | |
|---|---|---|---|---|
| ber.: | C 71,07% | H 6,71% | N 10,36% | S 11,86% |
| gef.: | C 71,13%, | H 6,72% | N 10,40% | S 11,83% |

Beispiel 21

1-Butyl-9-benzyl-7,8,9,10-tetrahydrothieno[3,2-e]-pyrido[4,3-b]indol

Die Verbindung entsteht analog der in Beispiel 5 beschriebenen aus 25,6 g 3-Butyl-5-hydrazinobenzothiophenhydrochlorid und 23 g 1-Benzyl--4-piperidon. Schmelzpunkt 127-128°C.

Ausbeute: 15 g (40% der Theorie)

$C_{24}H_{26}N_2S$ (374,554)

| | | | | |
|---|---|---|---|---|
| ber.: | C 76,96% | H 7,00% | N 7,48% | S 8,56% |
| gef.: | C 77,06% | H 7,12% | N 7,41% | S 8,57% |

Schmelzpunkt des Lactats: 138-139°C.

Beispiel 22

1-Phenyl-7,8,9,10-tetrahydrothieno[3,2-e]pyrido-[4,3-b]indol

28 g 5-Hydrazino-3-phenylbenzothiophenhydrochlorid werden in 300 ml Isopropanol gelöst und unter Inertgas mit 20 g 4-Piperidonhydrochloridmonohydrat vereinigt. Nach 3stündigem Kochen wird das Lösungsmittel abgedampft, der Rückstand in Isopropanol aufgenommen und unter Zugabe von Isopropyläther gefällt. Der Niederschlag wird abfiltriert, in Wasser aufgenommen; die Lösung wird mit Dichlormethan unterschichtet und mit NaOH-Lösung geschüttelt. Die abgetrennte organische Phase wird aufgearbeitet. Schmelzpunkt 228°C (Zers.).

Ausbeute: 13 g (43% der Theorie)

$C_{19}H_{16}N_2S$ (304,419)

ber.: C 74,96% H 5,30% N 9,20% S 10,53%
gef.: C 74,87% H 5,13% N 9,11% S 10,36%

Beispiel 23
9-sek.-Butyl-1-phenyl-7,8,9,10-tetrahydrothieno-[3,2-e]pyrido[4,3-b]indol

Die Verbindung entsteht analog der in Beispiel 15 beschriebenen aus 10 g 5-Hydrazino-3-phenylbenzothiophenhydrochlorid und 6,2 g 1-sek.-Butyl-4-piperidon. Die Reinigung erfolgt an Kieselgel mit Dichlormethan/Isopropanol/Ammoniak (10:2:0,05). Schmelzpunkt 167-168°C.
Ausbeute: 9 g (69% der Theorie)
$C_{23}H_{24}N_2S$ (360,52)
ber.: C 76,62% H 6,71% N 7,77% S 8,89%
gef.: C 76,48% H 6,69% N 7,82% S 8,78%
Schmelzpunkt des Hydrochlorids: 260°C (Zers.).

Beispiel 24
9-Benzyl-1-phenyl-7,8,9,10-tetrahydrothieno-[3,2-e]pyrido[4,3-b]indol

Die Verbindung entsteht aus 13,8 g 3-Phenyl-5-hydrazinobenzothiophenhydrochlorid und 12 g 1- Benzyl-4-piperidon, indem nach 1stündigem Kochen in Isopropanol/HCl unter $N_2$ eingedampft, das Konzentrat in Isopropanol aufgenommen, mit $NH_4OH$ alkalisch gemacht, extrahiert, wiederum eingedampft und an $Al_2O_3$ chromatographiert wird. Schmelzpunkt 115-116°C.
Ausbeute: 7,15 g (36% der Theorie)
$C_{26}H_{22}N_2S$ (394,54)
ber.: C 79,20% H 5,62% N 7,10% S 8,13%
gef.: C 79,25% H 5,58% N 7,15% S 8,01%

Beispiel 25
9-[4-(4-Fluorphenyl)-4-oxobutyl]-1-methyl-7,8,9,-10-tetrahydrothieno[3,2-e]pyrido[4,3-b]indol

24 g 1-Methyl-7,8,9,10-tetrahydrothieno[3,2-e]-pyrido[4,3-b]indol in 300 ml Dimethylformamid werden mit 30 g 4-Chlor-1-oxo-1-(4-fluorphenyl)-butanethylenketal unter $N_2$ auf 80° erhitzt. Nach 4 Stunden wird das Lösungsmittel abgedampft, der Rückstand mit Ammoniak alkalisch gemacht und mit Chloroform extrahiert. Der Extrakt wird gewaschen und getrocknet und das Lösungsmittel abgedampft. Der Rückstand wird in Methanol/HCl aufgenommen, 2 Stunden gerührt und 12 Stunden aufbewahrt. Das ausgefallene Hydrochlorid wird anschliessend mit Ammoniak/Chloroform in die Base verwandelt. Schmelzpunkt 186°C.
Ausbeute: 10 g (25% der Theorie)
$C_{24}H_{23}FN_2OS$ (406,53)
ber.: C 70,91% H 5,70% F 4,67% N 6,89% S 7,89%
gef.: C 70,73% H 5,79% F 4,6 % N 6,82% S 7,76%

Beispiel 26
9-[4,4-Bis-(4-fluorphenyl)butyl]-1-methyl-7,8,9,10-tetrahydrothieno[3,2-e]pyrido[4,3-b]indol

2,4 g 1-Methyl-7,8,9,10-tetrahydrothieno[3,2-e]-pyrido[4,3-b]indol wird in 50 ml Dimethylformamid und 10 ml Hexamethylphosphorsäuretriamid gelöst, mit 4,5 g 4,4-Bis-(4-fluorphenyl)butyljodid versetzt und unter $N_2$ 5 Stunden lang auf 80°C erwärmt. Anschliessend wird das Lösungsmittel abgedampft; der Rückstand in Chloroform aufgenommen, mit Wasser gewaschen, getrocknet, eingedampft, an $Al_2O_3$ mit Chloroform chromatografiert und aus Isopropanol rekristallisiert. Schmelzpunkt 158°C.
Ausbeute: 1,9 g (40% der Theorie)
$C_{30}H_{28}F_2N_2S$ (486,636)
ber.: C 74,04% H 5,79% F 7,8% N 5,75% S 6,58%
gef.: C 73,21% H 5,40% F 8,4% N 5,74% S 6,79%

Beispiel 27
9-(3-Dimethylaminopropyl)-1-methyl-7,8,9,10-tetrahydrothieno[3,2-e]pyrido[4,3-b]indol

10 g 1-Methyl-7,8,9,10-tetrahydrothieno[3,2-e]-pyrido[4,3-b]indol, gelöst in 200 ml Dimethylformamid bei 90°C, werden tropfenweise mit einer Lösung von 12 ml 3-Dimethylaminopropylchlorid in 50 ml Dimethylformamid 3 Stunden lang auf 90° erhitzt. Das Reaktionsgemisch wird abfiltriert, eingedampft und der Rückstand in Chloroform aufgenommen. Nach dem Waschen mit Wasser und Trocknen wird die organische Phase eingeengt, an $SiO_2$ mit Chloform/Methanol/$NH_4OH$ (7:3:0,1) chromatographiert und aus Isopropanol rekristallisiert. Schmelzpunkt 184°C.
Ausbeute: 5,1 g (38% der Theorie)
$C_{19}H_{25}N_3S$ (327,499)
ber.: C 69,68% H 7,70% N 12,83% S 9,79%
gef.: C 69,53% H 7,69% N 12,89% S 9,93%
Schmelzpunkt des Hydrochlorids: $>$ 350°C.

Beispiel 28
6-Propyl-7,8,9,10-tetrahydrothieno[3,2-e]pyrido-[4,3-b]indolmaleat

11,5 g 7,8,9,10-Tetrahydrothieno[3,2-e]pyrido-[4,3-b]indol werden in 20 ml Dimethylformamid gelöst, unter $N_2$ mit 2,5 g einer NaH-Dispersion (50%ig) versetzt und bis zur beendeten $H_2$-Entwicklung (ca. 30 Minuten) gerührt. Anschliessend werden bei 0°C 4,5 ml Propylbromid, gelöst in Dimethylformamid, zugetropft. Das Lösungsmittel wird abgedampft, der Rückstand in Ether aufgenommen und mit n NaOH gewaschen. Nach weiterem Waschen mit Wasser und Trocknen der organischen Phase wird eingedampft, die als gelbliches Öl zurückbleibende Base in Isopropanol aufgenommen und mit einer Lösung von 15 g Maleinsäure in Isopropanol versetzt. Schmelzpunkt 171°C.
Ausbeute: 5 g (25% der Theorie)
$C_{20}H_{22}N_2O_4S$ (386,48)
ber.: C 62,16% H 5,74% N 7,24%
gef.: C 62,13% H 5,50% N 7,29%

Beispiel 29
9-Methyl-1-phenyl-6-propyl-7,8,9,10-tetrahydrothieno[3,2-e]pyrido[4,3-b]indol

Die Verbindung entsteht analog der in Beispiel 28 beschriebenen aus 4,8 g 9-Methyl-1-phenyl-7,8,9,10-tetrahydrothieno[3,2-e]pyrido[4,3-b]-indol, NaH und 1,5 ml Propylbromid. Schmelzpunkt 156°C.
Ausbeute: 2,8 g (52% der Theorie)
$C_{23}H_{24}N_2S$ (360,527)
ber.: C 76,82% H 6,71% N 7,77% S 8,89%
gef.: C 76,68% H 6,84% N 7,67% S 8,87%

Beispiel 30
6,9-Dibenzyl-1-methyl-7,8,9,10-tetrahydrothieno-[3,2-e]pyrido[4,3-b]indol

Die Verbindung entsteht analog der in Beispiel 28 beschriebenen aus 3,3 g 9-Benzyl-1-methyl-7,8,9,10-tetrahydrothieno[3,2-e]pyrido[4,3-b]-indol und 2 ml Benzylbromid in 50 ml Hexamethylphosphorsäuretriamid in Gegenwart von NaH. Schmelzpunkt 206°C.
Ausbeute: 2 g (48% der Theorie).

Beispiel 31
6-(3-Dimethylaminopropyl)-1,9-dimethyl-7,8,9,10--tetrahydrothieno[3,2-e]pyrido[4,3-b]indoldihydrochlorid

4,0 g 1,9-Dimethyl-7,8,9,10-tetrahydrothieno-[3,2-e]pyrido[4,3-b]indol werden in 60 ml Dimethylformamid gelöst, unter Rühren 0,78 g 50%-ige NaH Dispersion zugefügt und nach 1 Stunde mit 2,4 g 3-Dimethylaminopropylchlorid versetzt. Nach 15stündigem Stehen wird das Lösungsmittel abgedampft, der Rückstand in Ethylacetat aufgenommen, gewaschen, getrocknet und mit Chloroform/Methanol (1:1) an $SiO_2$ gereinigt. Das Dihydrochlorid wird anschliessend mit Isopropylalkohol/HCl hergestellt. Schmelzpunkt 263°C.
Ausbeute: 3 g (46% der Theorie)
$C_{20}H_{27}N_3S$ . 2 HCl (414,456)
ber.: C 57,96% H 7,05% N 10,14% S 7,74% Cl 17,11%
gef.: C 58,35% H 7,35% N 9,69% S 7,52% Cl 16,69%

Beispiel 32
9-Benzyl-6-(3-dimethylaminopropyl)-1-methyl-7,-8,9,10-tetrahydrothieno[3,2-e]pyrido[4,3-b]indol

Die Verbindung entsteht aus 6,6 g 9-Benzyl-1--methyl-7,8,9,10-tetrahydrothieno[3,2-e]pyrido-[4,3-b]indol, 1,0 g 50%ige NaH-Dispersion und 3 ml 3-Dimethylaminopropylchlorid in 50 ml Hexamethylphosphorsäuretriamid, bei 20°C innerhalb von 2 Stunden. Es wird in der üblichen Weise aufgearbeitet. Die erhaltene Base schmilzt bei 109°C.
Ausbeute: 6,0 g (72% der Theorie)
$C_{26}H_{31}N_3S$ (417,624)
ber.: C 74,47% H 7,48% N 10,06% S 7,68%
gef.: C 74,81% H 7,47% N 10,19% S 7,80%
Schmelzpunkt des Dihydrochlorids: 238°C.

Beispiel 33
6-(3-Dimethylaminopropyl)-1-methyl-7,8,9,10--tetrahydrothieno[3,2-e]pyrido[4,3-b]indol

Die Verbindung entsteht analog der in Beispiel 31 beschriebenen aus 4,8 g 1-Methyl-7,8,-9,10-tetrahydrothieno[3,2-e]pyrido[4,3-b]indol, 1,0 g NaH-Dispersion und 2,4 ml 3-Dimethylaminopropylchlorid in Dimethylformamid. Schmelzpunkt 92°C.
Ausbeute: 1,4 g (22% der Theorie)
$C_{19}H_{25}N_3S$ (327,499)
ber.: C 69,68% H 7,69% N 12,83% S 9,79%
gef.: C 69,65% H 7,79% N 12,72% S 9,91%
Schmelzpunkt des Dihydrochlorids 193°C (Zers.).

Beispiel 34
6,9-Bis-(3-dimethylaminopropyl)-1-methyl-7,8,9,-10-tetrahydrothieno[3,2-e]pyrido[4,3-b]indoltrimaleat

Die Verbindung entsteht analog der in Beispiel 31 beschriebenen aus 5 g 9-(3-Dimethylaminopropyl)-1-methyl-7,8,9,10-tetrahydrothieno[3,2-e]-pyrido[4,3-b]indol, NaH und 3-Dimethylaminopropylchlorid in Dimethylformamid und anschliessender Umwandlung in das Maleat.
Schmelzpunkt 174°C.
Ausbeute: 7,9 g (68% der Theorie)
$C_{24}H_{36}N_4S$ .
3 $C_4H_4O_4$ (760,878)
ber.: C 56,83% H 6,39% N 7,36% S 4,21%
gef.: C 57,51% C 6,30% N 7,38% S 3,28%

Beispiel 35
1,9-Dimethyl-7,8,9,10-tetrahydrothieno[3,2-e]-pyrido[4,3-b]indol-$N_{(9)}$-Methojodid

5 g 1,9-Dimethyl-7,8,9,10-tetrahydrothieno[3,2-e]pyrido[4,3-b]indol werden in 200 ml Ethanol gelöst und mit 10 ml Methyljodid versetzt. Nach längerem Stehen kristallisiert das Endprodukt aus. Schmelzpunkt 280-285°C (Zers.).
Ausbeute: 6,5 g (84% der Theorie)
$C_{16}H_{19}JN_2S$ (398,320)
ber.: C 48,25% H 4,81% N 7,03%
gef.: C 48,39% H 4,87% N 6,95%

Beispiel 36
9-Methyl-1-ethyl-7,8,9,10-tetrahydrothieno[3,2-e]-pyridol[4,3-b]indol

6,0 g 3-Ethyl-5-hydrazinobenzothiophenhydrochlorid werden in 100 ml Wasser suspendiert, mit Dichlormethan unterschichtet und mit Natriumhydrogencarbonat unter Rühren versetzt. Nach 15 Minuten wird die organische Phase abgetrennt, mit Wasser gewaschen, über Natriumsulfat getrocknet, filtriert und zur Trockene eingedampft. Die Base wird in 60 ml Ethylenglykol aufgenommen, mit 4,0 g 1-Methyl-4-piperidon versetzt und nach 1stündigem Rühren bei Raumtemperatur 3 Stunden auf 165°C erhitzt. Man lässt abkühlen, gibt zu der noch warmen Lösung 60 ml Methanol hinzu und lässt auskristallisieren. Das Rohprodukt wird säulenchromatographisch an Kieselgel mit Chloroform/Methanol (8:2) gereinigt. Schmelzpunkt 181-182°C.
Ausbeute: 4,0 g (57% der Theorie).
$C_{16}H_{18}N_2S$ (270,402)
ber.: C 71,07% H 6,71% N 10,36% S 11,85%
gef.: C 70,95% H 6,72% N 10,29% S 11,59%

Beispiel 37
1,9-Diethyl-7,8,9,10-tetrahydrothieno[3,2-e]pyrido[4,3-b]indol

Die Verbindung entsteht analog der in Beispiel 36 beschriebenen aus 8,0 g 2-Ethyl-5-hydrazinobenzothiophenhydrochlorid und 6,0 g 1--Ethyl-4-piperidon durch thermische Cyclisierung in Ethylenglykol über 3 Stunden bei 165-170°C.

Die kalte Reaktionsmischung wird mit Wasser verdünnt und anschliessend zweimal mit Chloroform ausgeschüttelt. Das nach dem Trocknen und Abdampfen des Lösungsmittels erhaltene Rohprodukt wird an Kieselgel mit Chloroform/Methanol (8:2) chromatographisch gereinigt. Die farblose Substanz schmilzt bei 158-159°C.

Ausbeute: 5,4 g (55% der Theorie).

$C_{17}H_{20}N_2S$ (284,426)

| | | | |
|---|---|---|---|
| ber.: | C 71,79% | H 7,08% | N 9,85% |
| gef.: | C 71,79% | H 7,05% | N 9,67% |

Beispiel 38

1-Ethyl-9-isopropyl-7,8,9,10-tetrahydrothieno-[3,2-e]pyrido[4,3-b]indol

Die Verbindung entsteht analog der in Beispiel 37 beschriebenen aus 8,0 g 3-Ethyl-5-hydrazinobenzothiophenhydrochlorid und 7,0 g 1--Isopropyl-4-piperidon durch 3stündiges Erhitzen in Ethylenglykol. Schmelzpunkt 183°C.

Ausbeute: 4,0 g (38% der Theorie)

$C_{18}H_{22}N_2S$ (298,45)

| | | | |
|---|---|---|---|
| ber.: | C 72,44% | H 7,43% | N 9,38% |
| gef.: | C 72,42% | H 7,31% | N 9,39% |

Beispiel 39

9-Allyl-1-ethyl-7,8,9,10-tetrahydrothieno[3,2-e]-pyrido[4,3-b]indol

Die Verbindung entsteht analog der in Beispiel 37 beschriebenen aus 9,0 g 3-Ethyl-5-hydrazinobenzothiophenhydrochlorid und 8,0 g 1-Allyl-4-piperidon durch 3stündiges Erhitzen in Ethylenglykol. Schmelzpunkt 143-144°C.

Ausbeute: 5,0 g (43% der Theorie)

$C_{18}H_{20}N_2S$ (296,436)

| | | | |
|---|---|---|---|
| ber.: | C 72,93% | H 6,80% | N 9,45% |
| gef.: | C 72,89% | H 6,71% | N 9,43% |

Beispiel 40

1-Isopentyl-9-methyl-7,8,9,10-tetrahydrothieno-[3,2-e]pyrido[4,3-b]indol

27,0 g 5-Hydrazino-3-isopentylbenzothiophenhydrochlorid werden in 250 ml Diethylether suspendiert und unter Rühren mit 50 ml 2n NaOH versetzt. Nach 10 Minuten werden die Phasen getrennt, die Etherlösung mit Wasser gewaschen, über Natriumsulfat getrocknet, filtriert und eingedampft. Der Rückstand wird mit 200 ml Ethylenglykol und 14,8 ml 1-Methyl-4-piperidon versetzt und 1 Stunde auf 180°C erhitzt. Das nach dem Abkühlen erhaltene kristalline Produkt wird aus Isopropanol umkristallisiert.

Schmelzpunkt 184-185°C.

Ausbeute: 15,0 g (47% der Theorie).

$C_{19}H_{24}N_2S$ (312,483)

| | | | | |
|---|---|---|---|---|
| ber.: | C 73,03% | H 7,77% | N 8,96% | S 10,26% |
| gef.: | C 72,88% | H 7,73% | N 9,04% | S 10,36% |

Schmelzpunkt des Lactats: 195-196°C;

Schmelzpunkt des Dihydrogenphosphats: 261-263°C.

Beispiel 41

9-Ethyl-1-phenyl-7,8,9,10-tetrahydrothieno[3,2-e]-pyrido[4,3-b]indol

13,8 g 5-Hydrazino-3-phenylbenzothiophenhydrochlorid und 6,7 g 1-Ethyl-4-piperidon werden 1 Stunde in 60 ml abs. Isopropanol gerührt, dann mit der gleichen Menge chlorwasserstoffhaltigem Isopropanol versetzt und 4 Stunden unter Rückfluss gekocht. Nach dem Abdampfen des Lösungsmittels behandelt man den Rückstand mit n NaOH und extrahiert die Base mit Dichlormethan. Die aus Toluol umkristallisierte Substanz schmilzt bei 223°C unter Zersetzung.

Ausbeute: 8,2 g (49% der Theorie).

$C_{21}H_{20}N_2S$ (332,5)

| | | |
|---|---|---|
| ber.: | C 75,87% | H 6,06% |
| gef.: | C 75,84% | H 6,18% |

Schmelzpunkt des Lactats: 163-164°C.

Beispiel 42

9-Propyl-1-phenyl-7,8,9,10-tetrahydrothieno-[3,2-e]pyrido[4,3-b]-indol

Die Verbindung entsteht analog der in Beispiel 40 beschriebenen aus 13,8 g 5-Hydrazino-3-phenylbenzothiophenhydrochlorid und 7,5 g 1-Propyl-4-piperidon durch 4stündiges Kochen in 120 ml chlorwasserstoffhaltigem Isopropanol. Schmelzpunkt 187-188°C.

Ausbeute: 7,0 g (40% der Theorie).

$C_{22}H_{22}N_2S$ (346,5)

| | | | | |
|---|---|---|---|---|
| ber.: | C 76,26% | H 6,40% | N 8,08% | S 9,25% |
| gef.: | C 75,52% | H 6,37% | N 8,11% | S 9,21% |

Schmelzpunkt des Lactats: 185°C.

Beispiel 43

9-Isopropyl-1-phenyl-7,8,9,10-tetrahydrothieno-[3,2-e]pyrido[4,3-b]indol

Die Verbindung entsteht analog der in Beispiel 40 beschriebenen aus 13,8 g 5-Hydrazino--3-phenylbenzothiophenhydrochlorid und 7,5 g 1-Isopropyl-4-piperidon durch 4stündiges Kochen in 120 ml chlorwasserstoffhaltigem Isopropanol. Schmelzpunkt 204°C.

Ausbeute: 6,0 g (35% der Theorie).

$C_{22}H_{22}N_2S$ (346,5)

| | | | | |
|---|---|---|---|---|
| ber.: | C 76,26% | H 6,40% | N 8,06% | S 9,25% |
| gef.: | C 75,55% | H 6,42% | N 8,08% | S 9,21% |

Schmelzpunkt des Lactats: 205°C.

Beispiel 44

9-Phenethyl-1-phenyl-7,8,9,10-tetrahydrothieno-[3,2-e]pyrido[4,3-b]indol

28,0 g 5-Hydrazino-3-phenylbenzothiophenhydrochlorid lässt man bei Raumtemperatur in 500 ml Isopropanol mit 21,0 g N-(2-Phenethyl)-4-piperidon 2 Stunden reagieren. Nach dem Absaugen des Kristallbreis wird dieser in 150 ml n NaOH suspendiert und mit Dichlormethan extrahiert. Die organische Phase wird mit Wasser neutralgewaschen, abgedampft, der ölige Rückstand in 400 ml Ethylenglykol aufgenommen und 30 Minuten auf 190°C erhitzt. Die nach dem Abkühlen

auskristallisierte Substanz wird abgesaugt und aus Toluol umkristallisiert. Schmelzpunkt 220-221°C.

$C_{27}H_{24}N_2S$ (408,60)

| | | | | |
|---|---|---|---|---|
| ber.: | C 79,37% | H 5,92% | N 7,77% | S 7,84% |
| gef.: | C 79,49% | H 5,91% | N 7,67% | S 7,75% |

Analog zu den Beispielen 5, 12 und 15 wurden die in Tabelle 1 aufgeführten Wirkstoffe hergestellt.

In Analogie zu den Beispielen 26 bis 28 wurden die in Tabelle 2 zusammengestellten Verbindungen synthetisiert.

TABELLE I

| Beispiel | Ausgangsverbindung (II) 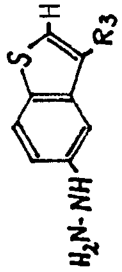 | Ausgangsverbindung (III) 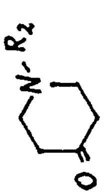 | Endverbindung (I) 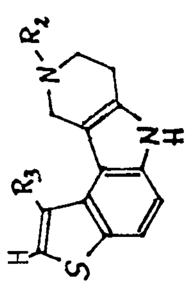 | | Bemerkungen |
|---|---|---|---|---|---|
| | $R_3$ | $R_2$ | Schmelzpunkt (°C) | Ausbeute an Base in % d.Th. | Reaktionsmedium Reaktionsdauer Temperatur |
| 45 | $-CH_2CH_2CH_2CH_3$ | $-CH_2CH_3$ | 140-141 (Base)<br>188-189 (Lactat) | 49 | Ethylenglykol; 140 Min.; 185°C |
| 46 | $-CH_2CH_2CH_2CH_3$ | $-CH_2CH_2CH_3$ | 126-127 (Base)<br>187 (Lactat) | 44 | Ethylenglykol; 40 Min.; 185°C |
| 47 | $-CH_2CH_3$ | -H | 215-216 (Base)<br>206 (Lactat) | 38 | Eisessig; 150 Min.; 40°C |
| 48 | $-CH_2CH_2CH_2CH_3$ | $-CH_2CH(CH_3)_2$ | 108 (Base)<br>170-171 (Lactat) | 32 | Ethylenglykol; 40 Min.; 185°C |
| 49 | $-CH_2CH_2CH_2CH_3$ | $-CH_2CH=CH_2$ | 128 (Base)<br>140 (Lactat) | 48 | Ethylenglykol; 45 Min.; 185°C |
| 50 | $-CH_2CH_2CH_2CH(CH_3)_2$ | -H | 182 (Base)<br>190-191 (Lactat) | 60 | Isopropanol/HCl; 120 Min.; Rückfluss |
| 51 | $-CH_2CH_2CH(CH_3)_2$ | $-CH_2CH=CH_2$ | 131 (Base)<br>156-158 (Lactat) | 53 | Isopropanol/HCl; 90 Min.; Rückfluss |
| 52 | $-CH_2CH_2CH_2CH(CH_3)_2$ | Cyclohexyl $-C_6H_{11}$ | 165 (Base)<br>173-175 (Lactat) | 60 | Isopropanol/HCl; 60 Min.; Rückfluss |
| 53 | $-CH_2CH_3$ | Phenyl $-C_6H_5$ | 181-182 (Base) | 49 | Eisessig; 60 Min.; 70°C |
| 54 | $-CH_2CH_2C_6H_5$ | $-CH_3$ | 181 (Lactat) | 45 | Isopropanol/HCl; 180 Min.; Rückfluss |
| 55 | $-CH_2CH_2C_6H_5$ | $-CH_2CH_2CH_3$ | 171 (Lactat) | 52 | Isopropanol/HCl; 150 Min.; Rückfluss |
| 56 | $-CH_3$ | $-CH_2CH_3$ | 201 (Base)<br>201 (Lactat) | 50 | Isopropanol/HCl; 30 Min.; Rückfluss |

TABELLE 2

| Beispiel | Ausgangsverbindung | | Endverbindung (I) | | | Bemerkungen |
|---|---|---|---|---|---|---|
| | 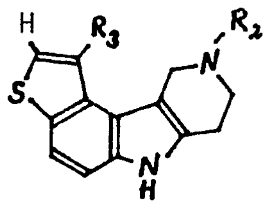 | | | 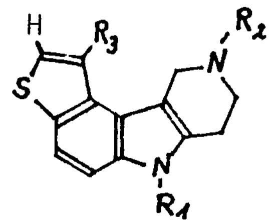 | | Reaktionsmedium<br>Alkylierungsmittel<br>Reaktionsdauer<br>Temperatur |
| | $R_2$ | $R_3$ | $R_1$ | Schmelzpunkt (°C) | Ausb. Base % d.Th. | |
| 57 | $-CH_3$ | 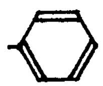 | $-CH_2CH_2CH(CH_3)_2$ | 147 (Base) | 73 | Dimethylformamid/NaH;<br>$R_1$-Br; 6 Stdn.; 20°C |
| 58 | $-CH_3$ | $-CH_2CH_2CH(CH_3)_2$ | $-CH_2CH_2$-O-$CH_3$ | 127-128 (Base) | 61 | Hexamethylphosphorsäuretriamid/NaH;<br>$R_1$-Cl; 10 Stdn.; 20°C |
| 59 | $-CH_3$ | $-CH_2CH_2CH(CH_3)_2$ | $-CH_2CH_2CH_2N(CH_3)_2$ | 238-240 (Zers.)<br>Dihydrochlorid | 55 | Hexamethylphosphorsäuretriamid/NaH;<br>$R_1$-Cl; 32 Stdn.; 20°C |
| 60 | H | $-CH_3$ | $R_1$ u. $R_2$ = $-CH_2$- 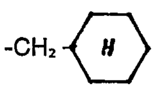 | 244 (Hydrochlorid) | 23 | Dimethylformamid/NaH;<br>$R_1$-Br; 2 Stdn.; 80°C |
| 61 | H | $-CH_3$ | $R_1$ = H;<br>$R_2$ = $-CH_2$- 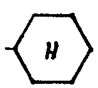 | 173 (Base) | 20 | Dimethylformamid (ohne NaH);<br>$R_1$-Br; 4 Stdn.; 80°C |

**Patentansprüche**

1. 7,8,9,10-Tetrahydrothieno[3,2-e]pyrido-[4,3-b]indole der allgemeinen Formel (I)

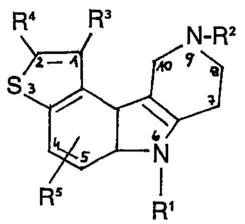

(I)

in welcher

$R^1$ für Wasserstoff, geradkettiges oder verzweigtes Alkyl mit 1 bis 4 Kohlenstoffatomen das gegebenenfalls durch Alkoxy mit 1 bis 2 Kohlenstoffatomen substituiert ist oder durch Alkylamino oder Dialkylaminoreste substituiert ist mit jeweils 1 bis 2 Kohlenstoffatomen pro Alkylrest oder für Phenylalkyl steht wobei der Phenylrest gegebenenfalls substituiert ist durch Halogen oder

für Phenyl steht, wobei der Phenylrest gegebenenfalls durch Alkyl oder Alkoxyreste mit 1 bis 2 Kohlenstoffatomen substituiert ist oder gegebenenfalls durch Halogen oder durch Hydroxyl oder Trifluormethyl substituiert ist, oder

für eine Carbonylgruppe steht, die durch Alkyl mit 1 bis 4 Kohlenstoffatomen oder Phenyl substituiert ist, wobei die Phenylgruppe gegebenenfalls durch Nitro, Halogen oder Amino substituiert ist,

oder für eine Cycloalkylalkylgruppe mit 3 bis 6 Kohlenstoffatomen im Cycloalkylring und 1 bis 3 Kohlenstoffatomen in der Alkylkette steht,

$R^2$ für Wasserstoff, geradkettiges oder verzweigtes oder cyclisches Alkyl mit bis zu 6 Kohlenstoffatomen, wobei dieser Alkylrest gegebenenfalls substituiert ist durch Alkoxy mit 1 bis 2 Kohlenstoffatomen oder durch Alkylamino oder Dialkylaminoreste mit jeweils 1 bis 2 Kohlenstoffatomen im Alkylteil, oder für Phenyl steht, wobei dieser Phenylrest gegebenenfalls substituiert ist durch Alkylreste mit jeweils 1 bis 2 Kohlenstoffatomen oder durch Alkoxy mit 1 bis 2 Kohlenstoffatomen oder durch Halogen oder Trifluormethyl, oder

für Benzyl oder Phenethyl steht wobei der Phenylring gegebenenfalls substituiert ist durch Halogen, oder für Alkenyl mit 1 bis zu 4 Kohlenstoffatomen steht, oder

für einen Alkoxycarbonylrest steht mit bis zu 4 Kohlenstoffatomen im Alkoxyrest, wobei dieser Alkoxyrest gegebenenfalls durch Dialkylaminogruppen mit jeweils 1 oder 2 Kohlenstoffatomen in der Alkylgruppe substituiert ist oder

für einen Benzoylalkylrest, wobei der Alkylrest bis zu 4 Kohlenstoffatome enthält und der Phenylrest gegebenenfalls durch Halogen substituiert ist, steht, oder

für einen Biphenylalkylrest steht mit 1 bis 4 Kohlenstoffatomen im Alkylrest, wobei der Phenylrest gegebenenfalls durch Halogen substituiert ist,

$R^3$ für Wasserstoff, geradkettiges oder verzweigtes Alkyl mit 1 bis 6 Kohlenstoffatomen, gegebenenfalls substituiert durch Halogen oder für einen gegebenenfalls durch Halogen oder Trifluormethyl substituierten Phenylrest steht,

$R^4$ für Wasserstoff, Alkyl mit 1 bis 4 Kohlenstoffatomen, insbesondere mit 1 bis 2 Kohlenstoffatomen, oder für eine Benzyloxycarbonylgruppe steht,

sowie gegebenenfalls mit Alkylhalogeniden gebildeten quaternären Salze oder ihre gegebenenfalls mit Basen oder Säuren gebildeten physiologisch verträglichen Salze.

2. Verbindungen der allgemeinen Formel I gemäss Anspruch 1 in welcher

$R^1$ für Wasserstoff, geradkettiges oder verzweigtes Alkyl mit 1 bis 4 Kohlenstoffatomen das gegebenenfalls durch Alkoxy mit 1 bis 2 Kohlenstoffatomen substituiert ist, für Benzyl oder Phenyl steht, welche gegebenenfalls durch Fluor oder Chlor substituiert sind,

$R^2$ für Wasserstoff, geradkettiges oder verzweigtes oder cyclisches Alkyl mit bis zu 6 Kohlenstoffatomen, Phenyl oder Benzyl steht, oder für einen 3-(p-Fluorbenzoyl)-propyl- oder 4,4-Bis-(4-fluorphenyl)-butylrest steht,

$R^3$ für Wasserstoff, Alkyl mit 1 bis 2 Kohlenstoffatomen, Benzyl oder Phenäthyl steht, wobei die Alkyl- und Phenylreste gegebenenfalls durch Fluor oder Chlor substituiert sind, und

$R^4$ für Wasserstoff oder Alkyl mit 1 bis 2 Kohlenstoffatomen steht.

3. Verfahren zur Herstellung von Verbindungen der allgemeinen Formel (I)

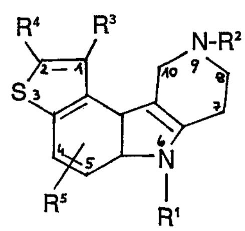

(I)

in welcher

$R^1$ für Wasserstoff, geradkettiges oder verzweigtes Alkyl mit 1 bis 4 Kohlenstoffatomen das gegebenenfalls durch Alkoxy mit 1 bis 2 Kohlenstoffatomen substituiert ist oder durch Alkylamino oder Dialkylaminoreste substituiert ist mit jeweils 1 bis 2 Kohlenstoffatomen pro Alkylrest oder für Phenylalkyl steht, wobei der Phenylrest gegebenenfalls substituiert ist durch Halogen oder

für Phenyl steht, wobei der Phenylrest gegebenenfalls durch Alkyl oder Alkoxyreste mit 1 bis 2 Kohlenstoffatomen substituiert ist oder gegebenenfalls durch Halogen oder durch Hydroxyl oder Trifluormethyl substituiert ist, oder

für eine Carbonylgruppe steht, die durch Alkyl mit 1 bis 4 Kohlenstoffatomen oder Phenyl substituiert ist, wobei die Phenylgruppe gegebenenfalls durch Nitro, Halogen oder Amino substituiert ist,

oder für eine Cycloalkylalkylgruppe steht, mit

3 bis 6 Kohlenstoffatomen im Cycloalkylring und bis 3 Kohlenstoffatomen in der Alkylkette,

$R^2$ für Wasserstoff, geradkettiges oder verzweigtes oder cyclisches Alkyl mit bis zu 6 Kohlenstoffatomen, wobei dieser Alkylrest gegebenenfalls substituiert ist durch Alkoxy mit 1 bis 2 Kohlenstoffatomen oder durch Alkylamino oder Dialkylaminoreste mit jeweils 1 bis 2 Kohlenstoffatomen im Alkylteil, oder für Phenyl steht, wobei der Phenylrest gegebenenfalls substituiert ist durch Alkylreste mit jeweils 1 bis 2 Kohlenstoffatomen oder durch Alkoxy mit 1 bis 2 Kohlenstoffatomen oder durch Halogen oder Trifluormethyl, oder

für Benzyl oder Phenethyl steht wobei der Phenylring gegebenenfalls substituiert ist durch Halogen oder für Alkenyl mit bis zu 4 Kohlenstoffatomen steht, oder

für einen Alkoxycarbonylrest steht mit bis zu 4 Kohlenstoffatomen im Alkoxyrest, wobei dieser Alkoxyrest gegebenenfalls durch Dialkylaminogruppen mit jeweils 1 oder 2 Kohlenstoffatomen in der Alkylgruppe substituiert ist oder

für einen Benzoylalkylrest, wobei der Alkylrest bis zu 4 Kohlenstoffatome enthält und der Phenylrest gegebenenfalls durch Halogen substituiert ist, steht, oder

für einen Biphenylalkylrest steht mit 1 bis 4 Kohlenstoffatomen im Alkylrest, wobei der Phenylrest gegebenenfalls durch Halogen substituiert ist,

$R^3$ für Wasserstoff, geradkettiges oder verzweigtes Alkyl mit 1 bis 6 Kohlenstoffatomen, gegebenenfalls substituiert durch Halogen oder für einen gegebenenfalls durch Halogen oder Trifluormethyl substituierten Phenylrest steht,

$R^4$ für Wasserstoff, Alkyl mit 1 bis 4 Kohlenstoffatomen, insbesondere mit 1 bis 2 Kohlenstoffatomen, oder für eine Benzyloxycarbonylgruppe steht,

sowie gegebenenfalls mit Alkylhalogeniden gebildeten quarternären Salze oder ihre gegebenenfalls mit Basen oder Säuren gebildeten physiologisch verträglichen Salze,

dadurch gekennzeichnet, dass man Hydrazinverbindungen der allgemeinen Formel (II)

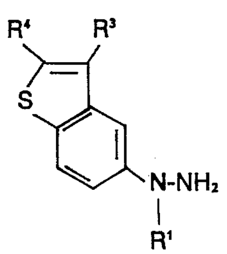

(II)

in welcher

$R^1$, $R^3$ und $R^4$ die oben angeführte Bedeutung haben, mit Piperidonen der allgemeinen Formel (III)

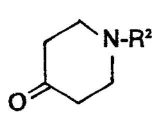

(III)

in welcher

$R^2$ die angeführte Bedeutung hat,

in Gegenwart von inerten organischen Lösungsmitteln bei Temperaturen zwischen 50 und 230°C und gegebenenfalls in Gegenwart von Kondensationsmitteln umsetzt und die so entstehenden Verbindungen der Formel (I) für den Fall, dass $R_1$ und $R_2$ Wasserstoffatome bedeuten, diese in bekannter Weise gegebenenfalls substituiert durch die für $R_1$ und $R_2$ obengenannten Reste und anschliessend die freien Verbindungen der Formel (I) gegebenenfalls in bekannter Weise in die quaternären oder die Additionssalze überführt.

4. Arzneimittel, enthaltend mindestens eine Verbindung gemäss Anspruch 1.

5. Verfahren zur Herstellung von Arzneimitteln, dadurch gekennzeichnet, dass man Verbindungen gemäss Anspruch 1 gegebenenfalls mit Hilfs- und Trägerstoffen in eine geeignete Applikationsform überführt.

6. Verbindungen der allgemeinen Formel (I) gemäss Anspruch 1 zur Verwendung bei der Bekämpfung von Erkrankungen des zentralen Nervensystems.

**Claims**

1. 7,8,9,10-Tetrahydrothieno[3,2-e]pyrido-[4,3-b]indoles of the general formula (I)

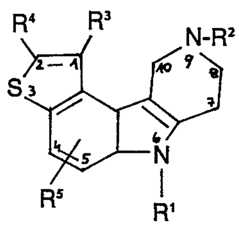

(I)

in which

$R^1$ represents hydrogen, straight-chain or branched alkyl with 1 to 4 carbon atoms which is optionally substituted by alkoxy with 1 to 2 carbon atoms or is substituted by alkylamino or dialkylamino radicals with in each case 1 to 2 carbon atoms per alkyl radical or represents phenylalkyl, the phenyl radical optionally being substituted by halogen or

represents phenyl, the phenyl radical optionally being substituted by alkyl or alkoxy radicals with 1 to 2 carbon atoms or optionally being substituted by halogen or by hydroxyl or trifluoromethyl, or

represents a carbonyl group, which is substituted by alkyl with 1 to 4 carbon atoms or phenyl, the phenyl group being optionally substituted by nitro, halogen or amino,

or represents a cycloalkylalkyl group with 3 to 6 carbon atoms in the cycloalkyl ring and 1 to 3 carbon atoms in the alkyl chain,

$R^2$ represents hydrogen, straight-chain or branched or cyclic alkyl with up to 6 carbon atoms, this alkyl radical optionally being substituted by alkoxy with 1 to 2 carbon atoms or

by alkylamino or dialkylamino radicals with in each case 1 to 2 carbon atoms in the alkyl part, or represents phenyl, this phenyl radical optionally being substituted by alkyl radicals with in each case 1 to 2 carbon atoms or by alkoxy with 1 to 2 carbon atoms or by halogen or trifluoromethyl,

or represents benzyl or phenethyl the phenyl ring optionally being substituted by halogen

or represents alkenyl with up to 4 carbon atoms,

or represents an alkoxycarbonyl radical with up to 4 carbon atoms in the alkoxy radical, this alkoxy radical optionally being substituted by dialkylamino groups with in each case 1 or 2 carbon atoms in the alkyl group

or represents a benzoylalkyl radical, the alkyl radical containing up to 4 carbon atoms and the phenyl radical optionally being substituted by halogen, or represents a biphenylalkyl radical with 1 to 4 carbon atoms in the alkyl radical, the phenyl radical optionally being substituted by halogen,

$R^3$ represents hydrogen, straight-chain or branched alkyl with 1 to 6 carbon atoms, optionally substituted by halogen, or phenyl radical optionally substituted by halogen or trifluoromethyl,

$R^4$ represents hydrogen, alkyl with 1 to 4 carbon atoms, in particular with 1 to 2 carbon atoms, or a benzyloxycarbonyl group,

and quaternary salts optionally formed with alkyl halides or their physiologically acceptable salts optionally formed with bases or acids.

2. Compounds of the general formula I according to Claim 1, in which

$R^1$ represents hydrogen, straight-chain or branched alkyl with 1 to 4 carbon atoms which is optionally substituted by alkoxy with 1 to 2 carbon atoms, or benzyl or phenyl, which are optionally substituted by fluorine or chlorine,

$R^2$ represents hydrogen, straight-chain or branched or cyclic alkyl with up to 6 carbon atoms, phenyl or benzyl, or represents a 3-(p--fluorobenzoyl)-propyl or 4,4-bis-(4-fluorophenyl)--butyl radical,

$R^3$ represents hydrogen, alkyl with 1 to 2 carbon atoms, benzyl or phenethyl, the alkyl and phenyl radicals optionally being substituted by fluorine or chlorine and

$R^4$ represents hydrogen or alkyl with 1 to 2 carbon atoms.

3. Process for the preparation of compounds of the general formula (I)

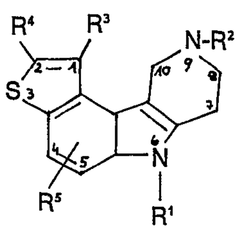

(I)

in which

$R^1$ represents hydrogen, straight-chain or branched alkyl with 1 to 4 carbon atoms which is optionally substituted by alkoxy with 1 to 2 carbon atoms or is substituted by alkylamino or dialkylamino radicals with in each case 1 to 2 carbon atoms per alkyl radical or represents phenylalkyl, the phenyl radical optionally being substituted by halogen or

represents phenyl, the phenyl radical optionally being substituted by alkyl or alkoxy radicals with 1 to 2 carbon atoms or optionally being substituted by halogen or by hydroxyl or trifluoromethyl, or

represents a carbonyl group, which is substituted by alkyl with 1 to 4 carbon atoms or phenyl, the phenyl group being optionally substituted by nitro, halogen or amino,

or represents a cycloalkylalkyl group with 3 to 6 carbon atoms in the cycloalkyl ring and 1 to 3 carbon atoms in the alkyl chain,

$R^2$ represents hydrogen, straight-chain or branched or cyclic alkyl with up to 6 carbon atoms, this alkyl radical optionally being substituted by alkoxy with 1 to 2 carbon atoms or by alkylamino or dialkylamino radicals with in each case 1 to 2 carbon atoms in the alkyl part, or represents phenyl, this phenyl radical optionally being substituted by alkyl radicals with in each case 1 to 2 carbon atoms or by alkoxy with 1 to 2 carbon atoms or by halogen or trifluoromethyl,

or represents benzyl or phenethyl, the phenyl ring optionally being substituted by halogen

or represents alkenyl with up to 4 carbon atoms,

or represents an alkoxycarbonyl radical with up to 4 carbon atoms in the alkoxy radical, this alkoxy radical optionally being substituted by dialkylamino groups with in each case 1 or 2 carbon atoms in the alkyl group

or represents a phenylcarbonyl radical, the alkyl radical containing up to 4 carbon atoms and the phenyl radical optionally being substituted by halogen,

or represents a benzoylalkyl radical with 1 to 4 carbon atoms in the alkyl radical, the phenyl radical optionally being substituted by halogen,

$R^3$ represents hydrogen, straight-chain or branched alkyl with 1 to 6 carbon atoms, optionally substituted by halogen, or a phenyl radical optionally substituted by halogen or trifluoromethyl,

$R^4$ represents hydrogen, alkyl with 1 to 4 carbon atoms, in particular with 1 to 2 carbon atoms, or a benzyloxycarbonyl group,

and quaternary salts optionally formed with alkyl halides or their physiologically acceptable salts optionally formed with bases or acids,

characterised in that hydrazine compounds of the general formula (II)

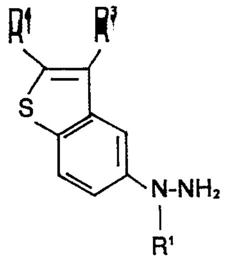

(II)

in which

$R^1$ und $R^3$ to $R^4$ have the meaning indicated above, are reacted with piperidones of the general formula (III)

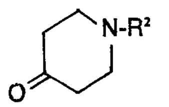

(III)

in which

$R^2$ has the meaning indicated,

in the presence of inert organic solvents at temperatures between 50 and 230°C and if appropriate in the presence of condensing agents, and, in the case where $R_1$ and $R_2$ in the compounds of the formula (I) thus formed denote hydrogen atoms, these compounds are optionally substituted by the radicals mentioned above for $R_1$ and $R_2$ in a known manner, and the free compounds of the formula (I) are then optionally converted into quaternary salts or addition salts in a known manner.

4. Medicaments containing at least one compound according to Claim 1.

5. Process for the preparation of medicaments, characterised in that compounds according to Claim 1 are converted into a suitable form of administration, if appropriate with auxiliaries and excipients.

6. Compounds of the general formula (I) according to Claim 1 for use in combating diseases of the central nervous system.

**Revendications**

1. 7,8,9,10-tétrahydrothiéno[3,2-e]pyrido-[4,3-b]indoles de formule générale (I):

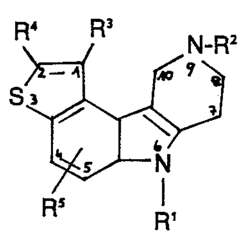

(I)

dans laquelle:

$R^1$ represente l'hydrogène, un groupe alkyle à chaîne droite ou ramifié ayant 1 à 4 atomes de carbone, qui est éventuellement substitué par un radical alkoxy ayant 1 ou 2 atomes de carbone ou substitué par un groupe alkylamino ou dialkylamino ayant chacun 1 ou 2 atomes de carbone par reste alkyle ou représente un groupe phénylalkyle, dont le reste phényle est éventuellement substitué par un halogène ou représente un reste phényle, ce reste phényle étant éventuellement substitué par des restes alkyle ou alkoxy ayant 1 ou 2 atomes de carbone ou substitué éventuellement par un halogène ou par un radical hydroxyle ou trifluorométhyle, ou représente un groupe carbonyle qui est substitué par un radical alkyle ayant 1 à 4 atomes de carbone ou par le groupe phényle, le groupe phényle étant éventuellement substitué par un radical nitro, un halogène ou un radical amino, ou représente un groupe cycloalkylalkyle ayant 3 à 6 atomes de carbone dans le noyau cycloalkyle et 1 à 3 atomes de carbone dans la chaîne alkylique,

$R^2$ représente l'hydrogène, un groupe alkyle à chaîne droite ou ramifié ou cyclique ayant jusqu'à 6 atomes de carbone, ce reste alkyle étant éventuellement substitué par un radical alkoxy ayant 1 ou 2 atomes de carbone ou par des restes alkylamino ou dialkylamino ayant 1 ou 2 atomes de carbone dans la partie alkylique, ou le reste phényle, ce reste phényle étant éventuellement substitué par des restes alkyle ayant chacun 1 ou 2 atomes de carbone ou alkoxy ayant 1 ou 2 atomes de carbone ou par un halogène ou le reste trifluorométhyle, ou

représente le reste benzyle ou phénéthyle, dont le noyau phénylique est éventuellement substitué par un halogène ou par un reste alcényle ayant jusqu'à 4 atomes des carbone, ou

représente un reste alkoxycarbonyle ayant jusqu'à 4 atomes de carbone dans le reste alkoxy, ce reste alkoxy étant éventuellement substitué par des groupes dialkylamino ayant chacun 1 ou 2 atomes de carbone dans le groupe alkyle, ou un reste benzoylalkyle, dont le reste alkyle contient jusqu'à 4 atomes de carbone et le reste phényle est éventuellement substitué par un halogène, ou représente un reste biphénylalkyle ayant 1 à 4 atomes de carbone dans le reste alkyle, le reste phényle étant éventuellement substitué par un halogène,

$R^3$ représente l'hydrogène, un groupe alkyle à chaîne droite ou ramifié ayant 1 à 6 atomes de carbone, éventuellement substitué par un halogène ou représente un reste phényle éventuellement substitué par un halogène ou un radical trifluorométhyle,

$R^4$ représente l'hydrogène, un reste alkyle ayant 1 à 4 atomes de carbone, notamment 1 ou 2 atomes de carbone, ou un groupe benzyloxycarbonyle,

ainsi que, le cas échéant, des sels quaternaires formés avec des halogénures d'alkyle ou leurs sels physiologiquement acceptables éventuellement formés avec des bases ou des acides.

2. Composés de la formule générale I suivant la revendication 1, dans laquelle

$R^1$ désigne l'hydrogène, un groupe alkyle à chaîne droite ou ramifié ayant 1 à 4 atomes de carbone, qui est éventuellement substitué par un reste alkoxy ayant 1 ou 2 atomes de carbone, ou représente le groupe benzyle ou phényle,

qui sont substitués éventuellement par du fluor ou du chlore,

$R^2$ représente l'hydrogène, un groupe alkyle à chaîne droite ou ramifié ou cyclique ayant jusqu'à 6 atomes de carbone, le groupe phényle ou le groupe benzyle, ou représente un reste 3-(p-fluorobenzoyl)-propyl- ou 4,4-bis-(4-fluorophényl)-butyle,

$R^3$ représente l'hydrogène, un reste alkyle ayant 1 ou 2 atomes de carbone, benzyle ou phénéthyle, les restes alkyle et phényle étant éventuellement substitués par du fluor ou du chlore, et

$R^4$ désigne l'hydrogène ou un reste alkyle ayant 1 ou 2 atomes de carbone.

3. Procédé de production de composés de formule générale (I):

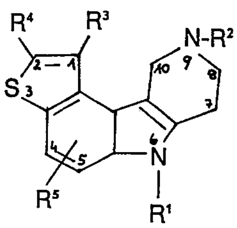

(I)

dans laquelle:

$R^1$ représente l'hydrogène, un groupe alkyle à chaîne droite ou ramifié ayant 1 à 4 atomes de carbone, qui est éventuellement substitué par un radical alkoxy ayant 1 ou 2 atomes de carbone ou substitué par un groupe alkylamino ou dialkylamino ayant chacun 1 ou 2 atomes de carbone par reste alkyle ou représente un groupe phénylalkyle, dont le reste phényle est éventuellement substitué par un halogène ou représente un reste phényle, le reste phényle étant éventuellement substitué par des restes alkyle ou alkoxy ayant 1 ou 2 atomes de carbone ou substitué éventuellement par un halogène ou par un radical hydroxyle ou trifluorométhyle, ou représente un groupe carbonyle qui est substitué par un radical alkyle ayant 1 à 4 atomes des carbone ou par le groupe phényle, le groupe phényle étant éventuellement substitué par un radical nitro, un halogène ou un radical amino, ou représente un groupe cycloalkylalkyle ayant 3 à 6 atomes de carbone dans le noyau cycloalkyle et 1 à 3 atomes de carbone dans la chaîne alkylique,

$R^2$ représente l'hydrogène, un groupe alkyle à chaîne droite ou ramifié ou cyclique ayant jusqu'à 6 atomes de carbone, ce reste alkyle étant éventuellement substitué par un radical alkoxy ayant 1 ou 2 atomes de carbone ou par des restes alkylamino ou dialkylamino ayant chacun 1 ou 2 atomes de carbone dans la partie alkylique, ou le reste phényle, ce reste phényle étant éventuellement substitué par des restes alkyle ayant chacun 1 ou 2 atomes de carbone ou alkoxy ayant 1 ou 2 atomes de carbone ou par un halogène ou le reste trifluorométhyle, ou

représente le reste benzyle ou phénéthyle, dont le noyau phénylique est éventuellement substitué par un halogène, ou un reste alcényle ayant jusqu'à 4 atomes de carbone, ou

représente un reste alkoxycarbonyle ayant jusqu'à 4 atomes de carbone dans le reste alkoxy, ce reste alkoxy étant éventuellement substitué par des groupes dialkylamino ayant chacun 1 ou 2 atomes de carbone dans le groupe alkyle, ou un reste phénylcarbonylalkyle dont le reste alkyle contient jusqu'à 4 atomes de carbone et le reste phényle est éventuellement substitué par un halogène, ou représente un reste benzoylalkyle ayant 1 à 4 atomes de carbone dans le reste alkyle, le reste phényle étant éventuellement substitué par un halogène,

$R^3$ représente l'hydrogène, un groupe alkyle à chaîne droite ou ramifié ayant 1 à 6 atomes de carbone, éventuellement substitué par un halogène ou représente un reste phényle éventuellement substitué par un halogène ou un radical trifluorométhyle,

$R^4$ représente l'hydrogène, un reste alkyle ayant 1 à 4 atomes de carbone, notamment 1 ou 2 atomes de carbone, ou un groupe benzyloxycarbonyle,

ainsi que des sels quaternaires éventuellement formés avec des halogénures d'alkyle ou de leurs sels physiologiquement acceptables éventuellement formés avec des bases ou des acides,

caractérisé en ce qu'on fait réagir des composés d'hydrazine de formule générale (II):

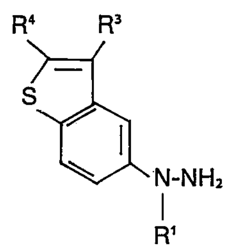

(II)

dans laquelle:

$R^1$, $R^3$ et $R^4$ ont la définition indiquée ci-dessus, avec des pipéridones de formule générale (III):

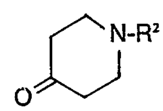

(III)

dans laquelle:

$R^2$ a la définition indiquée,

en présence de solvants organiques inertes, à des températures comprises entre 50 et 230°C et, le cas échéant en présence d'agents de condensation et au cas où, dans les composés ainsi obtenus de formule (I), $R_1$ et $R_2$ désignent des atomes d'hydrogène, ces derniers sont remplacés éventuellement d'une manière connue par les restes mentionnés ci-dessus pour $R_1$ et $R_2$, puis les composés libres de formule (I) sont éventuellement transformés d'une manière connue en les sels quaternaires ou les sels d'addition.

4. Médicament contenant au moins un composé suivant la revendication 1.

5. Procédé de production de médicaments, caractérisé en ce qu'on transforme des composés suivant la revendication 1 en une forme d'application correcte, éventuellement avec des adjuvants et des supports.

6. Composés de formule générale (I) suivant la revendication 1, destinés à être utilisés pour combattre de maladies du système nerveux central.